# EUROPEAN PATENT SPECIFICATION

(11) **EP 3 283 099 B1**
(45) Date of publication and mention of the grant of the patent: **02.03.2022**
(21) Application number: 16718244.3
(22) Date of filing: 07.04.2016
(51) Int. Cl.: C07K 16/36, A61K 38/48, A61K 39/395, A61P 7/04

(54) **COMBINATION THERAPY WITH COAGULATION FACTORS AND MULTISPECIFIC ANTIBODIES**
KOMBINATIONSTHERAPIE MIT GERINNUNGSFAKTOREN UND MULTISPEZIFISCHEN ANTIKÖRPERN
THÉRAPIE DE COMBINAISON AVEC DES ANTICORPS MULTISPÉCIFIQUES ET DES FACTEURS DE COAGULATION

(30) Priority: 17.04.2015 EP 15164045
(43) Date of publication of application: 21.02.2018
(73) Proprietor: F. Hoffmann-La Roche AG, 4070 Basel (CH)
(72) Inventor: CALATZIS, Andreas, 80469 München (DE); LECHNER, Katharina, 81371 Muenchen (DE)
(74) Representative: Burger, Alexander
(86) International application number: PCT/EP2016/057662
(87) International publication number: WO 2016/166014

(56) References cited:
- EP-A1- 1 688 488
- EP-A1- 2 644 698
- US-A1- 2005 209 149
- J. OLDENBURG ET AL: "Novel products for haemostasis - current status", HAEMOPHILIA, vol. 20, 25 May 2014 (2014-05-25), pages 23-28, XP055188542, ISSN: 1351-8216, DOI: 10.1111/hae.12428

## Description

### Field of the Invention

The invention relates to therapies for a patient with bleeding disorders, comprising the application of certain blood coagulation (clotting) factors in combination with antibodies.

### Background of the Invention

### Coagulation system

Any bigger organism has a blood circulation system, which delivers oxygen and nutritients to the different organs, and disposes carbon dioxide and wastes. However for the blood circulation system to function, injuries in the blood vessels have to be closed rapidly and effectively. This function is fulfilled by the blood coagulation system, which is a complex mechanism which allows the blood to form platelet aggregates and fibrin gels, which are able to close vascular injuries.

One mechanism involved in the blood coagulation system is the coagulation factor cascade, which is a series of serine proteases, which become serially activated and ultimately lead to the formation of thrombin, the central enzyme of the blood coagulation system. Thrombin is able to split fibrinogen to fibrin, which falls out, polymerizes into fibrin fibers, which form a fibrin clot. Thrombin is also activating co-factors, which accelerate its own generation (FV and FVIII), activates FXIII, a transglutaminase, which cross-links and thus stabilizes the fibrin clot, and thrombin is also a potent activator of the blood platelets.

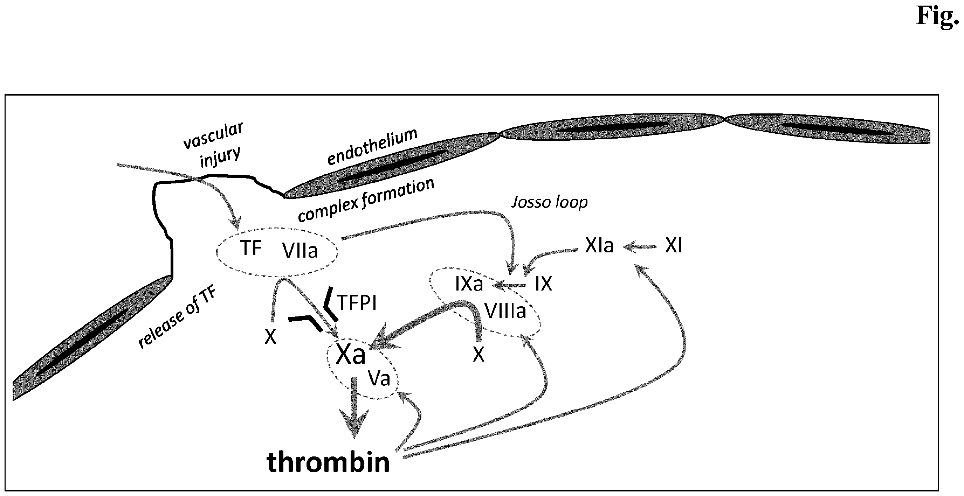

In the coagulation factor cascade two steps, which precede thrombin formation comprise enzymes (FXa and FIXa) which are extremely accelerated, when particular cofactors (FVa and FVIIIa respectively) are present in their active forms. As mentioned these cofactors are activated by thrombin, when it becomes available in the circulation in amounts sufficient to exceed the inhibitory capacity of the blood plasma. (see Figure 1)

### Bleeding

Bleeding is one of the most serious and significant manifestations in case of insufficent activation of the coagulation cascade and may occur from a local site or be systemic. Localized bleeding may be associated with lesions and may be further complicated by a defective haemostatic mechanism. Coagulation is inadequate in bleeding disorders, which may be caused by congenital coagulation disorders, acquired coagulation disorders, or hemorrhagic conditions induced by trauma. Congenital or acquired deficiencies of any of the coagulation factors may be associated with a hemorrhagic tendency.

### Blood coagulation factor deficiencies

A deficiency in coagulation factors can lead to bleeding complications. This deficiency can be due to hemodilution, i.e. transfusion of aqueous solutions, which do not contain coagulation factors or loss of platelets and/or coagulation factors due to low platelet numbers or increased coagulation activity and factor consumption.

Another reason for a deficiency of coagulation factors can be the application of vitamin K antagonists (coumadin or related compounds). Vitamin K antagonists inhibit the gamma carboxylation of the coagulation factors FVII, FX, FII and FIX, which renders these factors ineffective for the coagulation process.

Another reason for a deficiency of coagulation factors can be congenital genetic mutations, which lead to a functionally defective or diminished to no production of a particular coagulation factor. Due to the fact that most genes in the body are available in two copies (the paternal and maternal gene), mutations of coagulation factor encoding genes typically lead to no severe bleeding phenotype, as an activity of 50% of a coagulation factor is typically still sufficient for an adequate hemostasis of the patient. Therefore normally congenital coagulation factor deficiencies are quite rare, with two exceptions: hemophilia A and B.

### Hemophilia A and B

The coagulation factors FIX and FVIII are both encoded in the X chromosome. Males have only one X chromosome. Mutations in the FVIII or FIX genes can therefore lead to bleeding disorder phenotypes in males, as there is no genetic redundancy in this gene in males.

The prevalence of hemophilia A (FVIII deficiency) is approximately 1 in 5,000 live-born males or 1 out of every 10,000 live births. No racial differences have been reported, and the numbers of patients registered in 2012 in various regions included 4627 in Japan, 17,482 in North America, and 18,461 in the five major European nations (United Kingdom, France, Germany, Italy, and Spain).

The main bleeding sites are intra-articular, intramuscular, subcutaneous, intraoral, intracranial, gastrointestinal, and intranasal. Repeated intra-articular bleeding is a major factor that decreases health-related quality of life in patients with hemophilia A because it may progress to arthropathy and hemophilic arthropathy with walking disability, and joint replacement surgery may be necessary.

The severity of hemophilia A is classified in accordance with endogenous FVIII activity in the blood. Patients with FVIII activity less than 1% have severe disease, those with activity between 1% and 5% have moderate disease, and those with activity greater than 5% and less than 40% have mild disease. Patients who have severe hemophilia who do not comply with rigorous FVIII prophylaxis regimens or do not have access to FVIII products experience bleeding episodes several times a month, with a high frequency of spontaneous bleeding (annual bleeding rate of 30-40) which is much more frequently than in patients with moderate or mild disease.

Acquired hemophilia: Acquired hemophilia is a rare but potentially life-threatening bleeding disorder caused by the development of autoantibodies (inhibitors) directed against plasma coagulation factors, most frequently factor VIII (FVIII), but potentially also against von Willebrand factor, factors IX, V, XI, XII and XIII.

### Therapy of coagulation factor deficiencies

The therapy of coagulation factor deficiencies varies largely upon the underlying disorder.

In dilution coagulopathy all coagulation factors are missing. Therefore usually patients are treated with fresh frozen plasma, which contains all coagulation factors, including also the structural protein fibrinogen.

Bleedings due to the effect of vitamin K antagonists are typically treated with concentrated vitamin k dependent coagulation factors FII, FVII, FX and FIX. These concentrates are called prothrombin complex concentrates.

Single factor deficiencies are typically treated with the application of the respective factor, e.g. FVIII concentrates in hemophilia A, FIX concentrates in hemophilia B, FVII concentrate in FVII deficiency, etc.

A therapy with prothrombin complex concentrate will typically not improve the coagulation in hemophilia A patients, as these have normal concentrations in the factors FII, FVII, FX and FIX (as these patients lack FVIII). Also the indication for use of prothrombin complex concentrates is limited to the reversal of coagulation factor deficiencies of the vitamin k dependent factors.

An analysis of the effect of single coagulation factor activity on thrombin generation shows that thrombin generation is mostly compromised when the coagulation factor activity is below approx. 20% in most factors, and much less effect on thrombin generation is found at clotting factor levels between 20-100% (Al Dieri R, et al Thromb Haemost. 2002 Oct;88(4):576-82; see Figure 1 therein)

### Implications of procoagulant therapies using coagulation factors:

Bleedings caused by hemodilution are relatively rare events, typically occurring intra- or postoperatively or after traumatic bleedings. In these situations patients are typically treated surgically or in intensive care, where transfusion of plasma, coagulation factors and other drugs is routinely performed.

Bleedings caused by vitamin k antagonists are also relatively rare and can normally be managed by pausing the vitamin K antagonist or by application of vitamin K.

In contrast in patients with hemophilia A the bleeding disorder is a chronic condition and therefore often a prophylactic therapy is performed. Due to the short half-life of FVIII of only 8-19 hours for a prophylactic treatment i.v. infusions three times per week are necessary, which is cumbersome for the patients and impairs the quality of life. These i.v. applications are especially cumbersome in children with hemophilia A, which have a high need for the prophylactic treatment, due to the more frequent occurrence of smaller and larger injuries in children compared to adults.

Another problem of the substitution therapy with FVIII in hemophilia A patients, is that FVIII is a foreign protein for severe hemophilia A patients, and a considerably proportion of up to 30% of the patients form antibodies against factor FVIII ("inhibitors"), which make the therapy with FVIII unfeasible in many patients (because the transfused FVIII is rapidly inactivated in these patients by their anti-FVIII antibodies).

Certain therapeutic strategies exist to treat the coagulation disorder in patients with hemophilia A and inhibitors, namely the application of activated prothrombin complex concentrates (aPCCs, e.g. FEIBA^{®} by Baxter) or high levels of activated FVIIa (Novoseven^{®} by Novo Nordisk). Both strategies have disadvantages: Due to the application of activated coagulation factors, both agents can lead to thrombotic complications (e.g. Baudo et al, Blood 120(2012) 39-46, report a 3.6% rate of thrombosis in patients with acquired hemophilia treated with either rVIIa or aPCC). (see also Bui et al, J Thorac Cardiovasc Surg. 124(2002) 852-854, Chalwin et al; Eur J Cardiothorac Surg. 34(2008) 685-686, and Aledort; J Thromb Haemost. 2 (2004) 1700-1708)

In addition the agents do not contain FVIII and do not substitute the missing factor FVIII activity, and therefore can lead to an unstable hemostatic effect. rFVIIa products have a short blood half-life, and therefore require IV administration every 2-3 hours. Activated PCCs are applied approx. every 12h till the bleeding stops Oldenburg J. et al. (2014), Haemophilia 20, pages 23-28, reviews the current status of novel products for haemostasis and discloses the use of FIX as a medicament.

### Anti-factor IXa/X multispecific antibody ("FVIII mimicking antibodies" )

In order to overcome the limitations of the mentioned therapies for hemophilia A, anti-factor IXa/X bispecific antibodies have been developed which mimick the function of FVIII. These antibodies are bispecific and contain binding sites for FIX/FIXa as well as binding sites for FX. By this bispecific binding the antibody leads to an association of FIXa and FX, which significantly increases the enzymatic efficiency of FIXa in the absence of FVIII.

D1 discloses bispecific anti-factor IX/anti-factor X antibodies and use thereof for treatment of hemophilia.

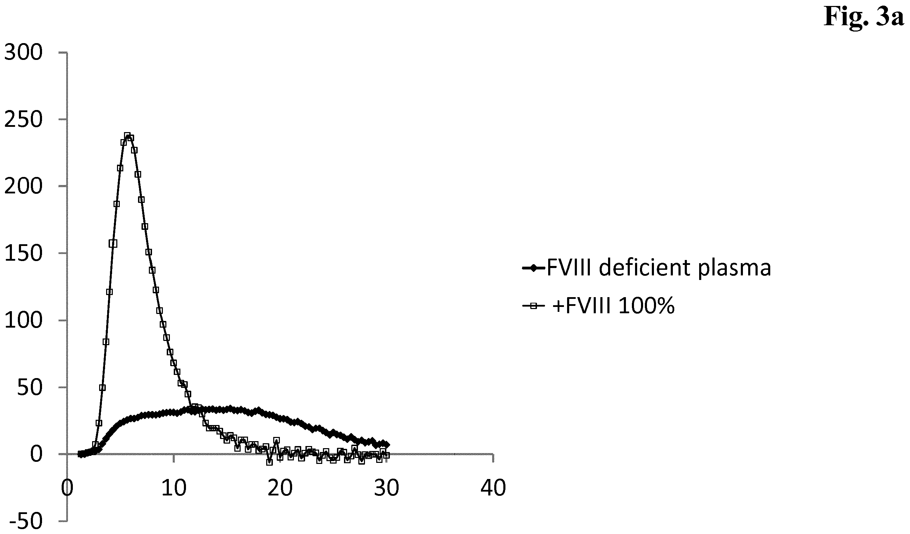

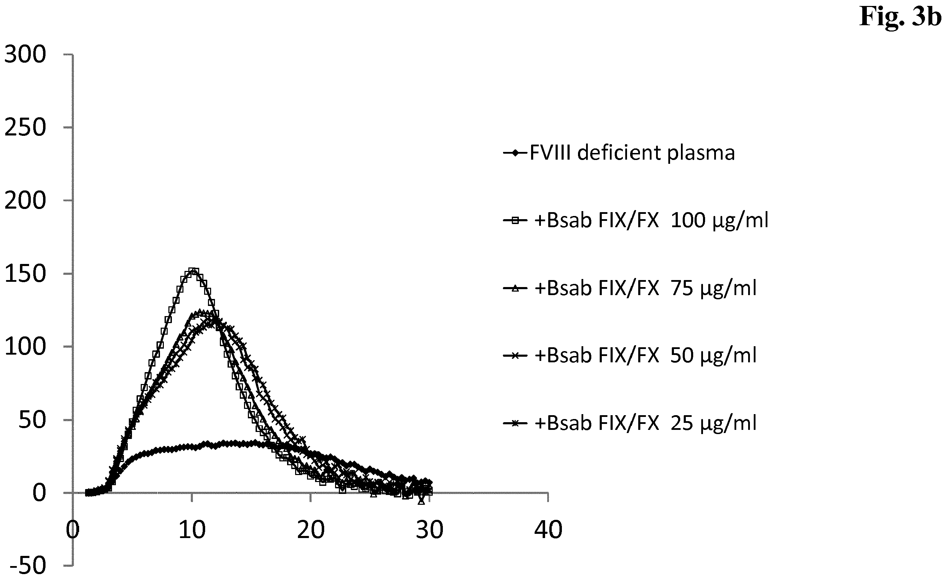

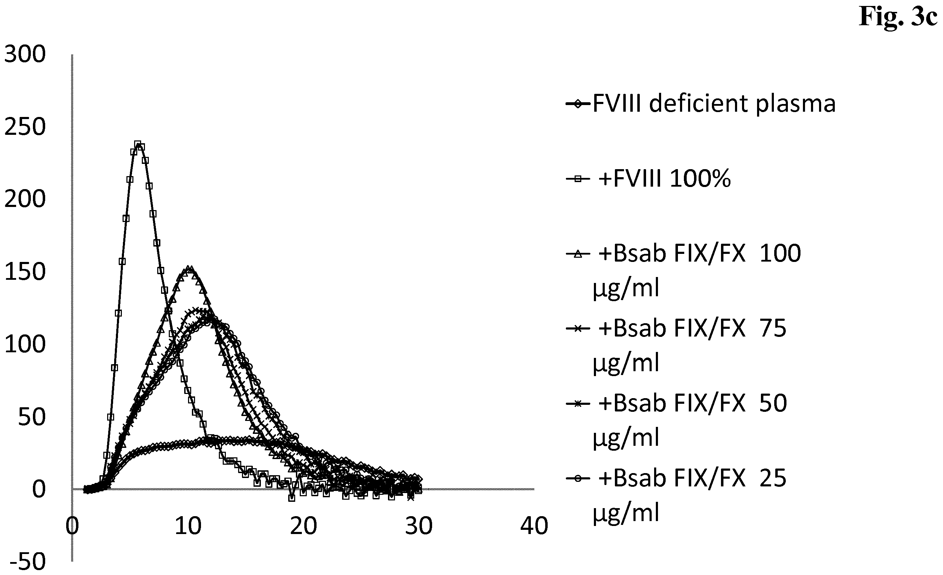

Bispecific Examples of anti-factor IXa/X multispecific antibodies are described e.g. US 2013/0330345, which increase the enzymatic activity of FIXa 5700 fold, which is approximately 10% of the acceleration, which is attained with a normal activity of FVIII in individuals without hemophilia. (see FIG 3 ) (Fay PJ. Activation of factor VIII and mechanisms of cofactor action. Blood Rev. 2004 Mar;18(1):1-15).

In clinical and preclinical studies performed using an anti-factor IXa/X bispecific antibody (ACE910; see A. Muto, et al , Blood, 124 (2014) 3165-3171)) in patients with hemophilia A with and without inhibitors it could be shown that this strategy is highly effective in preventing bleeding complications.

The use of such antibodies to treat hemophilia A has three distinct advantages compared to the use of FVIII:
Antibodies have long half-lifes of several weeks, which is approx. 40-50 times longer compared to the half-life of FVIII. This allows to perform a prophylactic treatment with applications of only 2x per month as compared to 3 x per week required for FVIII.

Antibodies can be given as s.c. injections, which is much easier and less cumbersome for the patient compared to the i.v. injections required for the application of FVIII. This is especially true for children that will be treated with these antibodies instead of FVIII infusions.

The application of ACE910 and similar antibodies does not cause inhibitor formation against FVIII.

As evidenced by the excellent efficacy of anti-factor IXa/X bispecific antibody ACE910 in the clinical studies (Abstracts 56th ASH Annual Meeting Program and Abstracts 691 Safety and Prophylactic Efficacy Profiles of ACE910, a Humanized Bispecific Antibody Mimicking the FVIII Cofactor Function, in Japanese Hemophilia A Patients Both without and with FVIII Inhibitors), the activity of anti-factor IXa/X bispecific antibodies in accelerating the enzymatic activity of FIX is sufficient for the prophylactic use of this drug in hemophilia A patients.

As mentioned the acceleration of FIXa activity by the presence of the anti-factor IXa/X bispecific antibody in the absence of FVIIIa is approx. 5700fold compared to the activity of FIXa alone, but about 90% weaker compared to the FIXa activity in the presence of FVIIIa. It is very likely that this moderate acceleration of the FIX activity by the anti-factor IXa/X bispecific antibody is beneficial in the long term prophylactic treatment, as this can lead to lower thrombogenic activity compared to the use of activated prothrombin complex concentrates or high doses of activated FVII.

### Unsolved problems:

While the therapy with FVIII mimicking antibodies has shown to be effective for use as prophylactic treatment of hemophilia A patients, it is imaginable that in certain patients higher procoagulant activities than the one provided by these antibodies alone are desirable. This could be the case e.g. following trauma or during large operations.

One option would be to increase the concentration of the FVIII mimicking antibody. However it has been shown that even increased concentrations of anti-factor IXa/X bispecific antibody ACE910 do not lead to the same FIXa activity as achieved with the FVIIIa activity of a healthy individual.

Obviously it would be imaginable to apply in these situations FVIII. This would not work in patients with FVIII inhibitors, as these would rapidly inactivate the transfused FVIII. In patients without inhibitors, the patient would be exposed to the risk of inhibitor formation.

Another strategy would be to apply in addition activated prothrombin complex concentrates or activated FVII. This would again expose the patient to the increased thrombotic risk associated with these compounds.

The therapy of coagulation factor deficiencies is usually based on the substitution of the missing coagulation factor. This means, if all factors are missing due to bleeding and hemodilution, the patient will typically receive all factors using fresh frozen plasma (FFP). If vitamin k dependent factors are missing due to a complication of anti-vitamin k therapy then the vitamin dependent factors are substituted using a prothrombin complex concentrate. If FVIII is missing (i.e. in hemophilia A), FVIII is transfused and if FIX is missing (i.e. in hemophilia B) FIX is given.

Inhibitor hemophilia A is a challenge, as it does not allow to substitute factor VIII, due to the fact that the antibodies against FVIII would rapidly inactivate the transfused factor, and also the transfusion can lead to an increase of the inhibitor levels in the patient, due to its immune response.

Here typically activated coagulation factors are transfused (activated prothrombin complex concentrates or high amounts of activated FVII). These activated factors restore the coagulation system activation even without the presence of the crucial co-factor FVIII. However there are also limitations, as these drugs come with a significant thrombotic risk and do not always provide a predictable and sustained hemostatic response.

FVIII mimicking antibodies are a novel strategy to restore the coagulation activation potential in hemophilia A patients with and without inhibitors. These new drugs mimick the effect of FVIII and increase the activity of FIX about 5,700 fold compared to the situation without FVIII. However the activity of FIXa reaches approximately 10% of the physiological activity with a normal level of FVIIIa. Most likely this is optimal for the prophylactic treatment of hemophilia A patients, as this moderate acceleration of FIXa activity is sufficient for the hemostatic response, without increasing the thrombotic risk.

However there is is still a need to further accelerate procoagulant activity of FVIII mimicking antibodies for specific situations, e.g. following trauma or during large operations.

### Summary of the Invention

The invention is defined in the appended claims and any other aspects set forth herein not falling within scope of the claims are for information only Any references in the description to methods of treatment refer to the compounds, pharmaceutical compositions and medicaments of the present invention for use in a method for treatment of the human (or animal) body by therapy.

The present invention provides a multispecific antibody (that functionally substitutes for blood coagulation factor VIII) which comprises a first antigen-binding site that binds to coagulation factor IX and/or activated coagulation factor IX and a second antigen- binding site that binds to coagulation factor X, for use in the treatment of hemophilia A, wherein the antibody is used in combination with a non-activaed coagulation factor IX.

The present invention provides a non-activated coagulation factor IX for use in the treatment of hemophilia A, wherein the coagulation factor IX is used in combination with a multispecific antibody (that functionally substitutes for blood coagulation factor VIII) which comprises a first antigen- binding site that binds to coagulation factor IX and/or activated coagulation factor IX and a second antigen- binding site that binds to coagulation factor X.

The present invention provides a combination of
i) a multispecific antibody (that functionally substitutes for blood coagulation factor VIII) which comprises a first antigen- binding site that binds to coagulation factor IX and/or activated coagulation factor IX and a second antigen- binding site that binds to coagulation factor X, and
ii) a non.activated coagulation factor IX,
for the use in the treatment of hemophilia A.

In one embodiment of the invention the patient suffering from a a) deficiency or b) malfunction of coagulation factor VIII, suffers from a congenital or acquired deficiency of coagulation factor VIII.

In one embodiment of the invention the deficiency is acquired by antibodies, other inhibitors, consumption or dilution.

The present invention provides a combination, antibody or use according to any one of the preceding embodiments,
a) for use in increasing the thrombin generation;
b) for use in increasing the thrombin generation at the site of vascular injury / at the site of tissue factor release;
c) for use accelerating of the thrombin generation/formation;
d) for use in increasing and accelerating the thrombin generation/formation;
e) for use in accelerating the thrombin generation/formation at the site of vascular injury / at the site of tissue factor release;
f) for use in enhancing blood coagulation;
g) for use in enhancing fibrin clot formation; and/or
h) for preventing and/or treating bleeding, diseases accompanying bleeding, diseases caused by bleeding, and the like.

The present invention provides a combination, antibody or use according to any one of the preceding embodiments
a) wherein there exists an increased bleeding risk,
b) during surgery or other invasive procedures, and/or
c) after vascular injury.

One embodiment of the invention is the combination, antibody or use described above, wherein in addition a) a coagulation factor II or b) a coagulation factor X, c) a coagulation factors II and X; or d) coagulation factors II, X and VII is used in the combination.

One embodiment of the invention is the combination, antibody or use described above, wherein coagulation factors IX is comprised in a prothrombin complex concentrates (PCC).

In one embodiment of the invention such prothrombin complex concentrates comprises FIX, FII, and FX.

In one embodiment of the invention such prothrombin complex concentrates comprises FIX, FII, FX and FVII..

In one embodiment of the invention the antibody described above is bispecific and the first antigen-binding site that binds to coagulation factor IX and/or activated coagulation factor IX comprises a H chain CDR 1, 2, and 3 amino acid sequences of SEQ ID NOs: 105, 106, and 107 (H chain CDRs of Q499), respectively,and a L chain CDR1, 2, and 3 amino acid sequences of SEQ ID NOs: 156, 157, and 158 (L chain CDR of L404), respectively, and the second antigen-binding site of comprises an H chain CDR 1, 2, and 3 amino acid sequences of SEQ ID NOs: 126, 127, and 128 (H chain CDRs of J327), respectively a L chain CDR1, 2, and 3 amino acid sequences of SEQ ID NOs: 156, 157, and 158 (L chain CDR of L404), respectively.

In one embodiment of the invention the antibody described above is a bispecific antibody (Q499-z121/J327-z119/L404-k), comprising a) a H chain consisting of the amino acid sequence of SEQ ID NO: 20, b) a H chain consisting of the amino acid sequence of SEQ ID NO: 25, and c) a (commonly shared) L chain of consisting of the amino acid sequence of ID NO: 32.

In one embodiment of the invention the multispecific antibody comprises a first polypeptide comprising a first antigen-binding site that binds to blood coagulation factor IX and/or activated blood coagulation factor IX and a third polypeptide comprising a third antigen-binding site that binds to blood coagulation factor IX and/or activated blood coagulation factor IX, as well as a second polypeptide comprising a second antigen-binding site that binds to blood coagulation factor X and a fourth polypeptide comprising a fourth antigen-binding site that binds to blood coagulation factor X. In one embodiment such mutltispecific antibody comprises a first polypeptide to fourth polypeptide wherein the first polypeptide and the third polypeptide each comprises an antigen-binding site of an H chain or L chain of an antibody against blood coagulation factor IX or activated blood coagulation factor IX, respectively; and the second polypeptide and the fourth polypeptide each comprises an antigen-binding site of an H chain or L chain of an antibody against blood coagulation factor X, respectively.

Surprisingly we found that increasing the coagulation factor IX (either alone or in combination with other coagulation factors like X, and/or II, e.g. also included in an PCC) over the physiological levels can significantly increase thrombin generation of hemophilia A patients treated with FVIII mimicking antibodies and is therefore useful for the treatment of hemophilia A. This is surprising, as one would not expect that increasing FIX levels over the physiological levels would increase thrombin generation especially in samples FVIII deficieny and hemophilia A (as FIX is so far only known for increasing the thrombin generation in FIX deficient plasma/patients suffering from hemophilia B).

Enhancing thrombin generation has multiple effects on the coagulation system as thrombin is the key enzyme of coagulation. Thrombin splits fibrinogen in fibrin and therefore results in fibrin clot formation, thrombin activates blood platelets. Therefore an increase in thrombin generation also results in an increased fibrin clot formation and platelet activation. In addition a regular thrombin formation also results in a lower fibrinolytic potential (by means of TAFI = thrombin activatable fibrinolysis inhibitor).

The advantages of the inventive method are manifold:
It allows to further extend the procoagulant activity of FVIII mimicking antibodies.

As only coagulation factors are transfused the thrombotic risk is low compared to the application of activated coagulation factors in activated PCCs and FVIIa. As none of the factors is related to FVIII, there is no risk of auto-antibody formation against FVIII by this treatment to hemophilia A patients.

Therefore by the present invention the valuable properties of anti-factor IXa/X bispecific antibodies (FVIII mimicking antibodies) like favorable pharmacokinetics, excellent activity in FVIII inhibitor patients, superior application route (s.c. vs. i.v.) are further supplemented by means to further increase the procoagulant effect of the medication. During the continued monotherapy with factor IXa/X bispecific antibody a high safety margin due to lower thrombin generation (compared to a FVIII treatment) can be achieved. The present invention is therefore especially useful for the (temporary) increased thrombin generation for certain incidents: surgery, acute trauma, etc. Due to the relatively short halflife of the coagulation factor IX (25h) (compared to the factor IXa/X bispecific antibody halflife (ca. 4-5 weeks after s.c. injection) used in combination with the antibody the effect of increased thrombin generation can be limited to certain time periods for the incidents mentioned above (without causing unnecessary thrombosis risk during longterm treatment).

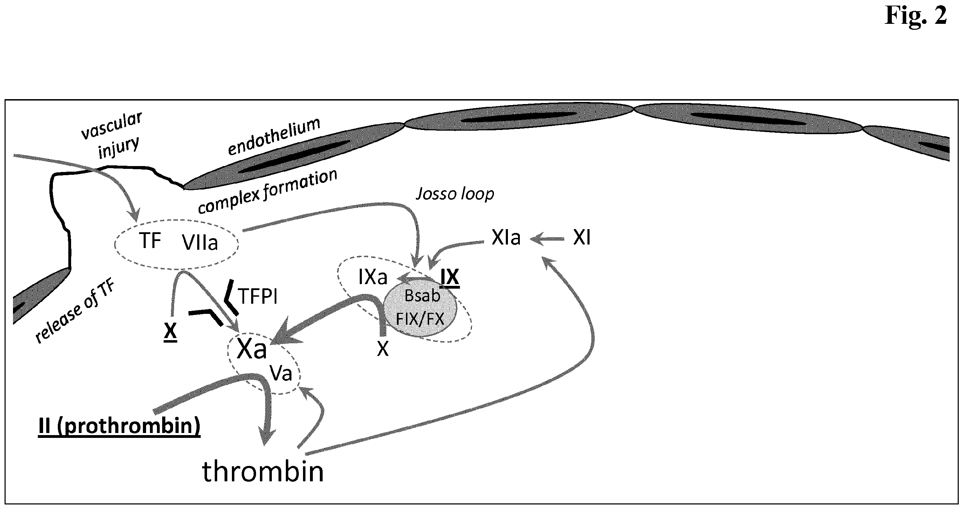

The combination of a anti-FX/FIXa antibody with non-activated clotting factors (FIX alone or in combination with one or several of the factors VII, X and II) does not add to the patient any form of an active coagulation enzyme. Only when tissue factor is released at the site of a vascular injury, thrombin generation can occur. (see Figure 2- exemplary scheme of the combination of the present invention)

In contrast the therapy with active clotting factors can impede the localization of the coagulation process at the site of the vascular injury which is shown by the significant thrombosis rate of approx. 3% (Baudo et al, Blood 120(2012) 39-46).

Therefore the invention allows an increased procoagulant activity of patients treated with FVIII mimicking antibodies, which is not based on the application of FVIII or activated coagulation factors.

Surprisingly we found that the application of the vitamin K dependent factors FIX, and additionally e.g. FX and FII, increases the thrombin generation (and therefore the procoagulant activity) of hemophilia A plasma treated with factor IXa/X bispecific antibody, even though the sample contains already normal levels of these factors. Also the application of FIX alone increases the thrombin generation of patient samples containing factor IXa/X bispecific antibody.

Another advantage of the inventive method is that the coagulation factors applied (FII, FIX and FX) have relatively long half-lifes in the circulation (~65h, ~25h, ~40h) compared to FVIIa (2.5h) and FVIII (12 h).

Another use of the combination of a factor IXa/X bispecific antibody with a coagulation factors FIX, FX and / or FII might also be benefitial in other situations which require a procoagulant treatment, such as bleeding due to directly or indirectly acting anticoagulants, surgery in patients with coagulopathies, or patients experiencing bleeding complications unrelated to a FVIII deficiency.

One aspect of the present invention are compositions which contain coagulation factors FIX, and/or and/or FII, which can be derived from plasma donations, or produced using recombinant protein production.

One embodiment of the invention is the combination, antibody or use as described herein, wherein the FIX is administered in an amount of 10 U - 200 U FIX / kg body weight in a patient with hemophilia A treated with anti-factor IXa/X bispecific antibody. In one embodiment additionally the FX is administered in an amount of 10 U - 200 U FX / kg body weight, preferably 50-200 U FIX/kg body weight.

One embodiment of the invention is the combination, antibody or use as described herein, wherein the FIX is administered in an amount of 10 U - 200 U FIX / kg body weight in a patient with hemophilia A treated with anti-factor IXa/X bispecific antibody. In one embodiment additionally the FII is administered in an amount of 10 U - 200 U FII / kg body weight.

One embodiment of the invention is the combination, antibody or use as described herein, wherein the FIX is administered in an amount of 10 U - 200 U FIX / kg body weight in a patient with hemophilia A treated with anti-factor IXa/X bispecific antibody. In one embodiment additionally the FII and FX are administered in an amount of 10 U - 200 U FII / kg body weight and 10 U - 200 U FX / kg body weight.

One embodiment of the invention is the combination, antibody or use as described herein, wherein the prothrombin complex (PCC) is administered in amount of 10 U - 200 U PCC / kg body weight in a patient with hemophilia A treated with anti-factor IXa/X bispecific antibody.

### Description of the Figures

**Figure 1** Schematic representation of the blood coagulation system: The figure shows a cross section of a blood vessel. The vessel wall are covered by endothelial cells, which have anti-throbmotic properties. When a vascular injury occurs, the endothial cell layer is disrupted and subendothelial cells are exposed to the blood. By this tissue factor (TF) is released, which is a transmembranal protein of subendothelial cells with strong procoagulant activity. TF forms a complex with clotting factor FVIIa, which then activates FX to FXa and FIX to FIXa. The direct activation of FX by the TF-FVIIa complex is inhibited by the tissue factor pathway inhibitor (TFPI), while the activity of factors Xa and XIa is slow as long as the co-factors FV and FVIII are not present in their active form. Once free thrombin is formed, it activates FV and FVIII to their active forms and the velocity of thrombin generation is greatly accelerated. However in hemophilia A FVIII is missing and therefore the velocity of thrombin generation stays very low which results in bleeding complications.
**Figure 2****:** Schematic representation of the combined application of the bispecific anti-FX/FIXa antibody (Bsab FIX/FX) with factor IX or factor IX in combination with FX and / or FII is shown. underlined: coagulation factors which increase the procoagulant activity of the Bsab FIX/FX
**Figures 3a to 3c****:** Comparative effects of anti-FX/FIXa antibody addition versus FVIII addition on thrombin generation in plasma samples which are deficient in coagulation factor VIII ( as model for diseases characterized by a deficiency or malfunction of coagulation factor VIII like e.g. hemophilia A) x-axis: time [minutes] y-axis: thrombin [nM]
**Figure 3a****:** The addition of FVIII leads to a rapid thrombin generation, and in total to a 7-fold higher thrombin generation as compared to the sample lacking FVIII.
**Figure 3b****:** Also the addition of Bsab FIX/FX leads to a significant increase of the thrombin generation which 3.4fold - 4.4 fold higher as compared to the sample lacking FVIII
**Figure 3c****:** Comparing the thrombin generation of samples with the supplementation of FVIII or Bsab FIX/FX,
**Figures 4a to 4b****:** Comparative effects of anti-FX/FIXa antibody in combination with FIX versus FVIII on thrombin generation in plasma samples which are deficient in coagulation factor VIII (as model for diseases characterized by a deficiency or malfunction of coagulation factor VIII like e.g. hemophilia A) x-axis: time [minutes] y-axis: thrombin [nM]

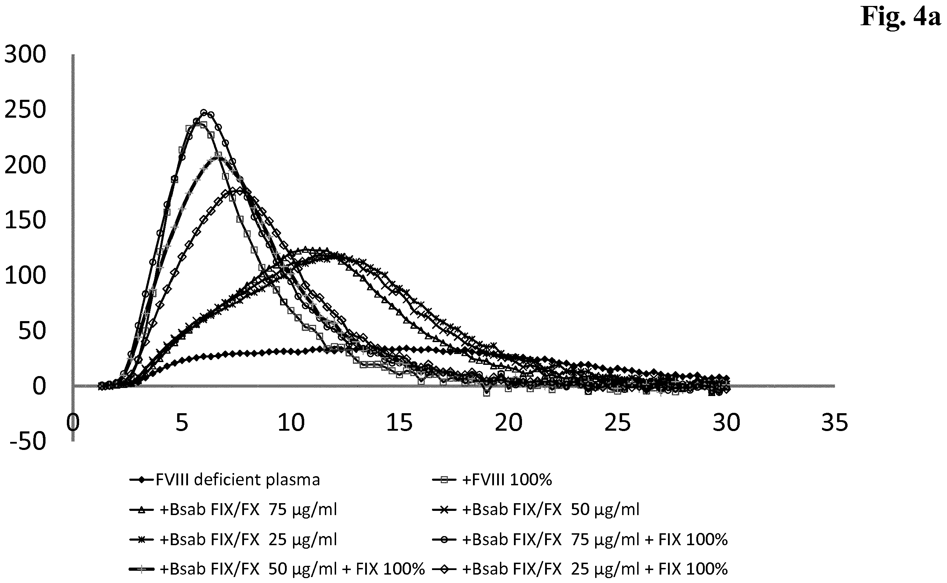

**Figure 4a****:** Combination/Addition of FIX to FVIII deficient plasma treated with Bsab FIX/FX: The addition of FIX (100%) to FVIII deficient plasma treated with Bsab FIX/FX leads to a significant increase of thrombin generation. In addition as shown in the diagram the time to peak was significantly shortened by the addition of FIX, i.e. thrombin generation was not only increased, but the thrombin generation was also accelerated. As seen in the diagram both the peak thrombin generation as well as the time to peak of the samples treated with Bsab FIX/FX matched the sample with the 100% FVIII.

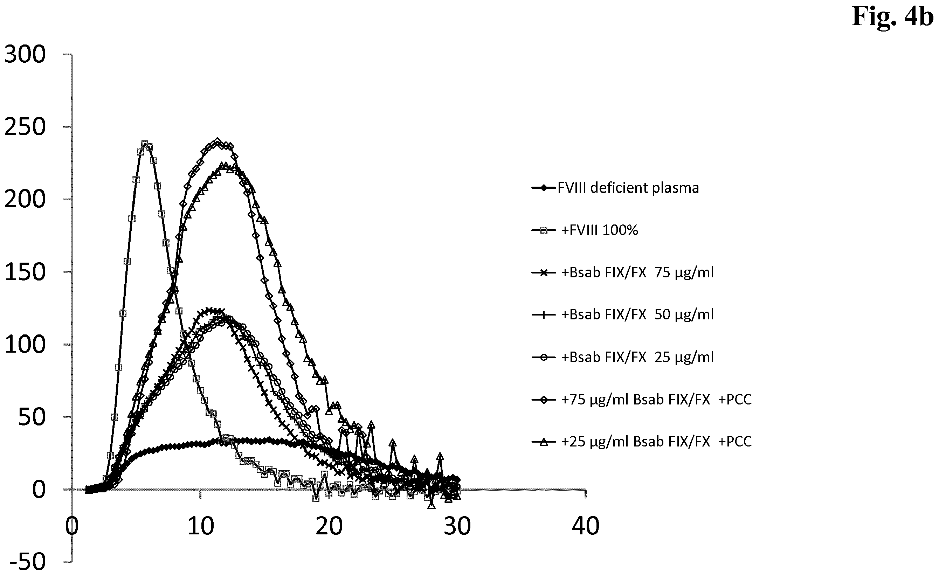

**Figure 4b****:** Combination/Addition of prothrombin complex concentrate (PCC) which comprise FIX to FVIII deficient plasma treated with Bsab FIX/FX: The addition of PCC (1 U/ml) to FVIII deficient plasma treated with Bsab FIX/FX lead to a significant increase of thrombin generation. In the sample with 75 µg Bsab FIX/FX /ml this resulted to a 94% increase of thrombin generation. In the sample with 25 µg RO5534262/ml a 90% increase of thrombin generation was found. In both cases the peak thrombin generation was similar for the Bsab FIX/FX treated samples with the addition of PCC compared to the FVIII supplemented sample.

### Detailed Description of the Invention

The invention is defined in the appended claims and any other aspects set forth herein not falling within scope of the claims are for information only.The present invention provides a multispecific antibody (that functionally substitutes for blood coagulation factor VIII) which comprises a first antigen-binding site that binds to coagulation factor IX and/or activated coagulation factor IX and a second antigen- binding site that binds to coagulation factor X, for use in the treatment of hemophilia A, wherein the antibody is used in combination with a non-activated coagulation factor IX.

The present invention provides a non-activated coagulation factor IX for use in the treatment of hemophilia A, wherein the coagulation factor IX is used in combination with a multispecific antibody (that functionally substitutes for blood coagulation factor VIII) which comprises a first antigen- binding site that binds to coagulation factor IX and/or activated coagulation factor IX and a second antigen- binding site that binds to coagulation factor X.

The present invention provides a combination of
i) a multispecific antibody (that functionally substitutes for blood coagulation factor VIII) which comprises a first antigen- binding site that binds to coagulation factor IX and/or activated coagulation factor IX and a second antigen- binding site that binds to coagulation factor X, and
ii) a non-activated coagulation factor IX,
for the use in the treatment of hemophilia A.

In one embodiment of the invention the patient suffering from a a) deficiency or b) malfunction of coagulation factor VIII, suffers from a congenital or acquired deficiency of coagulation factor VIII.

In one embodiment of the invention the deficiency is acquired by antibodies, other inhibitors, consumption or dilution.

The present invention provides a combination, antibody or use according to any one of the preceding embodiments,
a) for use in increasing the thrombin generation;
b) for use in increasing the thrombin generation at the site of vascular injury / at the site of tissue factor release;
c) for use accelerating of the thrombin generation/formation;
d) for use in increasing and accelerating the thrombin generation/formation;
e) for use in accelerating the thrombin generation/formation at the site of vascular injury / at the site of tissue factor release;
f) for use in enhancing blood coagulation;
g) for use in enhancing fibrin clot formation; and/or
h) for preventing and/or treating bleeding, diseases accompanying bleeding, diseases caused by bleeding, and the like.

The present invention provides a combination, antibody or use according to any one of the preceding embodiments
a) wherein there exists an increased bleeding risk,
b) during surgery or other invasive procedures, and/or
c) after vascular injury.

One embodiment of the invention is the combination, antibody or use described above, wherein in addition a) a coagulation factor II or b) a coagulation factor X, c) a coagulation factors II and X; or d) coagulation factors II, X and VII is used in the combination..

One embodiment of the invention is the combination, antibody or use described above, wherein coagulation factors IX is comprised in a prothrombin complex concentrates (PCC).

In one embodiment of the invention such prothrombin complex concentrates comprises FIX, FII, and FX.

In one embodiment of the invention such prothrombin complex concentrates comprises FIX, FII, FX and FVII..

In one embodiment of the invention the antibody described above is bispecific and the first antigen-binding site that binds to coagulation factor IX and/or activated coagulation factor IX comprises a H chain CDR 1, 2, and 3 amino acid sequences of SEQ ID NOs: 105, 106, and 107 (H chain CDRs of Q499) ), respectively,and a L chain CDR1, 2, and 3 amino acid sequences of SEQ ID NOs: 156, 157, and 158 (L chain CDR of L404), respectively.and the second antigen-binding site of comprises an H chain CDR 1, 2, and 3 amino acid sequences of SEQ ID NOs: 126, 127, and 128 (H chain CDRs of J327), respectively a L chain CDR1, 2, and 3 amino acid sequences of SEQ ID NOs: 156, 157, and 158 (L chain CDR of L404), respectively.

In one embodiment of the invention the antibody described above is a bispecific antibody (Q499-z121/J327-z119/L404-k), comprising a) a H chain consisting of the amino acid sequence of SEQ ID NO: 20, b) a H chain consisting of the amino acid sequence of SEQ ID NO: 25, and c) a (commonly shared) L chain of consisting of the amino acid sequence of ID NO: 32.

In one embodiment of the invention the multispecific antibody comprises a first polypeptide comprising a first antigen-binding site that binds to blood coagulation factor IX and/or activated blood coagulation factor IX and a third polypeptide comprising a third antigen-binding site that binds to blood coagulation factor IX and/or activated blood coagulation factor IX, as well as a second polypeptide comprising a second antigen-binding site that binds to blood coagulation factor X and a fourth polypeptide comprising a fourth antigen-binding site that binds to blood coagulation factor X. In one embodiment such mutltispecific antibody comprises a first polypeptide to fourth polypeptide wherein the first polypeptide and the third polypeptide each comprises an antigen-binding site of an H chain or L chain of an antibody against blood coagulation factor IX or activated blood coagulation factor IX, respectively; and the second polypeptide and the fourth polypeptide each comprises an antigen-binding site of an H chain or L chain of an antibody against blood coagulation factor X, respectively.

In one embodiment of the invention the antigen-binding site of the first polypeptide comprises an antigen-binding site which comprises H chain CDRs consisting of any one of the amino acid sequences selected from the following (a1) to (a11), or an antigen-binding site functionally equivalent thereto, and the antigen-binding site of the second polypeptide comprises an antigen-binding site which comprises H chain CDRs consisting of any one of the amino acid sequences selected from the following (b1) to (b11), or an antigen-binding site functionally equivalent thereto: (a1) an antigen-binding site comprising an H chain CDR 1, 2, and 3 amino acid sequences of SEQ ID NOs: 75, 76, and 77 (H chain CDRs of Q1), respectively; (a2) an antigen-binding site comprising an H chain CDR 1, 2, and 3 amino acid sequences of SEQ ID NOs: 78, 79, and 80 (H chain CDRs of Q31), respectively; (a3) an antigen-binding site comprising an H chain CDR 1, 2, and 3 amino acid sequences of SEQ ID NOs: 81, 82, and 83 (H chain CDRs of Q64), respectively; (a4) an antigen-binding site comprising an H chain CDR 1, 2, and 3 amino acid sequences of SEQ ID NOs: 84, 85, and 86 (H chain CDRs of Q85), respectively; (a5) an antigen-binding site comprising the H chain CDR 1, 2, and 3 amino acid sequences of SEQ ID NOs: 87, 88, and 89 (H chain CDRs of Q153), respectively; (a6) an antigen-binding site comprising an H chain CDR 1, 2, and 3 amino acid sequences of SEQ ID NOs: 90, 91, and 92 (H chain CDRs of Q354), respectively; (a7) an antigen-binding site comprising the H chain CDR 1, 2, and 3 amino acid sequences of SEQ ID NOs: 93, 94, and 95 (H chain CDRs of Q360), respectively; (a8) an antigen-binding site comprising the of H chain CDR 1, 2, and 3 amino acid sequences of SEQ ID NOs: 96, 97, and 98 (H chain CDRs of Q405), respectively; (a9) an antigen-binding site comprising an H chain CDR 1, 2, and 3 amino acid sequences of SEQ ID NOs: 99, 100, and 101 (H chain CDRs of Q458), respectively; (a10) an antigen-binding site comprising an H chain CDR 1, 2, and 3 amino acid sequences of SEQ ID NOs: 102, 103, and 104 (H chain CDRs of Q460), respectively; (a11) an antigen-binding site comprising an H chain CDR 1, 2, and 3 amino acid sequences of SEQ ID NOs: 105, 106, and 107 (H chain CDRs of Q499), respectively; (b1) an antigen-binding site comprising an H chain CDR 1, 2, and 3 amino acid sequences of SEQ ID NOs: 108, 109, and 110 (H chain CDRs of J232), respectively; (b2) an antigen-binding site comprising an H chain CDR 1, 2, and 3 amino acid sequences of SEQ ID NOs: 111, 112, and 113 (H chain CDRs of J259), respectively; (b3) an antigen-binding site comprising an H chain CDR 1, 2, and 3 amino acid sequences of SEQ ID NOs: 114, 115, and 116 (H chain CDRs of J268), respectively; (b4) an antigen-binding site comprising an H chain CDR 1, 2, and 3 amino acid sequences of SEQ ID NOs: 117, 118, and 119 (H chain CDRs of J300), respectively; (b5) an antigen-binding site comprising an H chain CDR 1, 2, and 3 amino acid sequences of SEQ ID NOs: 120, 121, and 122 (H chain CDRs of J321), respectively; (b6) an antigen-binding site comprising the H chain CDR 1, 2, and 3 amino acid sequences of SEQ ID NOs: 123, 124, and 125 (H chain CDRs of J326), respectively; (b7) an antigen-binding site comprising an H chain CDR 1, 2, and 3 amino acid sequences of SEQ ID NOs: 126, 127, and 128 (H chain CDRs of J327), respectively; (b8) an antigen-binding site comprising an H chain CDR 1, 2, and 3 amino acid sequences of SEQ ID NOs: 129, 130, and 131 (H chain CDRs of J339), respectively; (b9) an antigen-binding site comprising an H chain CDR 1, 2, and 3 amino acid sequences of SEQ ID NOs: 132, 133, and 134 (H chain CDRs of J344), respectively; (b10) an antigen-binding site comprising an H chain CDR 1, 2, and 3 amino acid sequences of SEQ ID NOs: 135, 136, and 137 (H chain CDRs of J346), respectively; and (b11) an antigen-binding site comprising an H chain CDR 1, 2, and 3 amino acid sequences of SEQ ID NOs: 174, 175, and 176 (H chain CDRs of J142), respectively.

In one embodiment of the invention the antigen-binding site of the first polypeptide comprises an antigen-binding site which comprises an H chain variable region consisting of any one of the amino acid sequences selected from the following (a1) to (a11), or an antigen-binding site functionally equivalent thereto, and the antigen-binding site of the second polypeptide comprises an antigen-binding site which comprises an H chain variable region consisting of any one of the amino acid sequences selected from the following (b1) to (b11), or an antigen-binding site functionally equivalent thereto: (a1) an antigen-binding site comprising an H chain variable region amino acid sequence of SEQ ID NO: 35 (H chain variable region of Q1); (a2) an antigen-binding site comprising an H chain variable region amino acid sequence of SEQ ID NO: 36 (H chain variable region of Q31); (a3) an antigen-binding site comprising an H chain variable region amino acid sequence of SEQ ID NO: 37 (H chain variable region of Q1); (a4) an antigen-binding site comprising an H chain variable region amino acid sequence of SEQ ID NO: 38 (H chain variable region of Q85); (a5) an antigen-binding site comprising an H chain variable region amino acid sequence of SEQ ID NO: 39 (H chain variable region of Q153); (a6) an antigen-binding site comprising an H chain variable region amino acid sequence of SEQ ID NO: 40 (H chain variable region of Q354); (a7) an antigen-binding site comprising an H chain variable region amino acid sequence of SEQ ID NO: 41 (H chain variable region of Q360); (a8) an antigen-binding site comprising an H chain variable region amino acid sequence of SEQ ID NO: 42 (H chain variable region of Q405); (a9) an antigen-binding site comprising an H chain variable region amino acid sequence of SEQ ID NO: 43 (H chain variable region of Q458); (a10) an antigen-binding site comprising an H chain variable region amino acid sequence of SEQ ID NO: 44 (H chain variable region of Q460); (a11) an antigen-binding site comprising an H chain variable region amino acid sequence of SEQ ID NO: 45 (H chain variable region of Q499); (b1) an antigen-binding site comprising an H chain variable region amino acid sequence of SEQ ID NO: 46 (H chain variable region of J232); (b2) an antigen-binding site comprising an H chain variable region amino acid sequence of SEQ ID NO: 47 (H chain variable region of J259); (b3) an antigen-binding site comprising an H chain variable region amino acid sequence of SEQ ID NO: 48 (H chain variable region of J268); (b4) an antigen-binding site comprising an H chain variable region amino acid sequence of SEQ ID NO: 49 (H chain variable region of J300); (b5) an antigen-binding site comprising an H chain variable region amino acid sequence of SEQ ID NO: 50 (H chain variable region of J321); (b6) an antigen-binding site comprising an H chain variable region amino acid sequence of SEQ ID NO: 51 (H chain variable region of J326); (b7) an antigen-binding site comprising an H chain variable region amino acid sequence of SEQ ID NO: 52 (H chain variable region of J327); (b8) an antigen-binding site comprising an H chain variable region amino acid sequence of SEQ ID NO: 53 (H chain variable region of J339); (b9) an antigen-binding site comprising an H chain variable region amino acid sequence of SEQ ID NO: 54 (H chain variable region of J344); (b10) an antigen-binding site comprising an H chain variable region amino acid sequence of SEQ ID NO: 55 (H chain variable region of J346); and (b11) an antigen-binding site comprising an H chain variable region amino acid sequence of SEQ ID NO: 172 (H chain variable region of J142).

In one embodiment of the invention the antigen-binding sites included in the third polypeptide and the fourth polypeptide comprise an antigen-binding site which comprises L chain CDRs consisting of any one of the amino acid sequences selected from the following (c1 to (c10), or an antigen-binding site functionally equivalent thereto: (c1) an antigen-binding site comprising an L chain CDR1, 2, and 3 amino acid sequences of SEQ ID NOs: 138, 139, and 140 (L chain CDR of L2), respectively; (c2) an antigen-binding site comprising an L chain CDR1, 2, and 3 amino acid sequences of SEQ ID NOs: 141, 142, and 143 (L chain CDR of L45), respectively; (c3) an antigen-binding site comprising an L chain CDR1, 2, and 3 amino acid sequences of SEQ ID NOs: 144, 145, and 146 (L chain CDR of L248), respectively; (c4) an antigen-binding site comprising an L chain CDR1, 2, and 3 amino acid sequences of SEQ ID NOs: 147, 148, and 149 (L chain CDR of L324), respectively; (c5) an antigen-binding site comprising an L chain CDR1, 2, and 3 amino acid sequences of SEQ ID NOs: 150, 151, and 152 (L chain CDR of L3 34), respectively; (c6) an antigen-binding site comprising an L chain CDR1, 2, and 3 amino acid sequences of SEQ ID NOs: 153, 154, and 155 (L chain CDR of L377), respectively; (c7) an antigen-binding site comprising an L chain CDR1, 2, and 3 amino acid sequences of SEQ ID NOs: 156, 157, and 158 (L chain CDR of L404), respectively; (c8) an antigen-binding site comprising an L chain CDR1, 2, and 3 amino acid sequences of SEQ ID NOs: 159, 160, and 161 (L chain CDR of L406), respectively; (c9) an antigen-binding site comprising an L chain CDR1, 2, and 3 amino acid sequences of SEQ ID NOs: 137, 138, and 139 (L chain CDR of L408), respectively; and (c10) an antigen-binding site comprising an L chain CDR1, 2, and 3 amino acid sequences of SEQ ID NOs: 177, 178, and 179 (L chain CDR of L180), respectively.

In one embodiment of the invention the antigen-binding sites included in the third polypeptide and the fourth polypeptide comprise an antigen-binding site which comprises an L chain variable region consisting of any one of the amino acid sequences selected from the following (c1) to (c10), or an antigen-binding site functionally equivalent thereto: (c1) an antigen-binding site comprising an L chain variable region amino acid sequence of SEQ ID NO: 56 (L chain variable region of L2); (c2) an antigen-binding site comprising an L chain variable region amino acid sequence of SEQ ID NO: 57 (L chain variable region of L45); (c3) an antigen-binding site comprising an L chain variable region amino acid sequence of SEQ ID NO: 58 (L chain variable region of L248); (c4) an antigen-binding site comprising an L chain variable region amino acid sequence of SEQ ID NO: 59 (L chain variable region of L324); (c5) an antigen-binding site comprising an L chain variable region amino acid sequence of SEQ ID NO: 60 (L chain variable region of L334); (c6) an antigen-binding site comprising an L chain variable region amino acid sequence of SEQ ID NO: 61 (L chain variable region of L377); (c7) an antigen-binding site comprising an L chain variable region amino acid sequence of SEQ ID NO: 62 (L chain variable region of L404); (c8) an antigen-binding site comprising an L chain variable region amino acid sequence of SEQ ID NO: 63 (L chain variable region of L406); (c9) an antigen-binding site comprising an L chain variable region amino acid sequence of SEQ ID NO: 64 (L chain variable region of L408); and (c10) an antigen-binding site comprising an L chain variable region amino acid sequence of SEQ ID NO: 173 (L chain variable region of L180).

In one embodiment of the invention the first and second polypeptides further comprise an antibody H chain constant region, and the third and fourth polypeptides comprise an antibody L chain constant region.

In one embodiment of the invention the first and second polypeptides comprise an antibody H chain constant region, and the third and fourth polypeptides comprise an antibody L chain constant region, and wherein the third polypeptide and the fourth polypeptide are a commonly shared L chain.

In one embodiment of the invention the first polypeptide comprises any one antibody H chain selected from the following (a1) to (a14), the second polypeptide comprises any one antibody H chain selected from the following (b1) to (b12), and the third polypeptide and the fourth polypeptide comprise any one antibody L chain selected from the following (c1) to (c10): (a1) an antibody H chain consisting of the amino acid sequence of SEQ ID NO: 1 (Q1-G4k); (a2) an antibody H chain consisting of the amino acid sequence of SEQ ID NO: 2 (Q31-z7); (a3) an antibody H chain consisting of the amino acid sequence of SEQ ID NO: 3 (Q64-z55); (a4) an antibody H chain consisting of the amino acid sequence of SEQ ID NO: 10 (Q64-z7); (a5) an antibody H chain consisting of the amino acid sequence of SEQ ID NO: 11 (Q85-G4k); (a6) an antibody H chain consisting of the amino acid sequence of SEQ ID NO: 12 (Q153-G4k); (a7) an antibody H chain consisting of the amino acid sequence of SEQ ID NO: 13 (Q354-z106); (a8) an antibody H chain consisting of the amino acid sequence of SEQ ID NO: 14 (Q360-G4k); (a9) an antibody H chain consisting of the amino acid sequence of SEQ ID NO: 15 (Q360-z118); (a10) an antibody H chain consisting of the amino acid sequence of SEQ ID NO: 16 (Q405-G4k); (a11) an antibody H chain consisting of the amino acid sequence of SEQ ID NO: 17 (Q458-z106); (a12) an antibody H chain consisting of the amino acid sequence of SEQ ID NO: 18 (Q460-z121); (a13) an antibody H chain consisting of the amino acid sequence of SEQ ID NO: 19 (Q499-z118); (a14) an antibody H chain consisting of the amino acid sequence of SEQ ID NO: 20 (Q499-z121); (b1) an antibody H chain consisting of the amino acid sequence of SEQ ID NO: 4 (J268-G4h); (b2) an antibody H chain consisting of the amino acid sequence of SEQ ID NO: 5 (J321-G4h); (b3) an antibody H chain consisting of the amino acid sequence of SEQ ID NO: 6 (J326-z107); (b4) an antibody H chain consisting of the amino acid sequence of SEQ ID NO: 7 (J344-z107); (b5) an antibody H chain consisting of the amino acid sequence of SEQ ID NO: 21 (J232-G4h); (b6) an antibody H chain consisting of the amino acid sequence of SEQ ID NO: 22 (J259-z107); (b7) an antibody H chain consisting of the amino acid sequence of SEQ ID NO: 23 (J300-z107); (b8) an antibody H chain consisting of the amino acid sequence of SEQ ID NO: 24 (J327-z107); (b9) an antibody H chain consisting of the amino acid sequence of SEQ ID NO: 25 (J327-z119); (b10) an antibody H chain consisting of the amino acid sequence of SEQ ID NO: 26 (J339-z119); (b11) an antibody H chain consisting of the amino acid sequence of SEQ ID NO: 27 (J346-z107); (b12) an antibody H chain consisting of the amino acid sequence of SEQ ID NO: 170 (J142-G4h); (c1) an antibody L chain consisting of the amino acid sequence of SEQ ID NO: 8 (L2-k); (c2) an antibody L chain consisting of the amino acid sequence of SEQ ID NO: 9 (L45-k); (c3) an antibody L chain consisting of the amino acid sequence of SEQ ID NO: 28 (L248-k); (c4) an antibody L chain consisting of the amino acid sequence of SEQ ID NO: 29 (L324-k); (c5) an antibody L chain consisting of the amino acid sequence of SEQ ID NO: 30 (L334-k); (c6) an antibody L chain consisting of the amino acid sequence of SEQ ID NO: 31 (L377-k); (c7) an antibody L chain consisting of the amino acid sequence of SEQ ID NO: 32 (L404-k); (c8) an antibody L chain consisting of the amino acid sequence of SEQ ID NO: 33 (L406-k); (c9) an antibody L chain consisting of the amino acid sequence of SEQ ID NO: 34 (L408-k); and (c10) an antibody L chain consisting of the amino acid sequence of SEQ ID NO: 171 (L180-k).

In one embodiment of the invention the antibody described above is a bispecific antibody of any one of the following (a) to (u):
(a) a bispecific antibody (Q1-G4k/J268-G4h/L45-k), wherein the first polypeptide is an H chain consisting of the amino acid sequence of SEQ ID NO: 1, the second polypeptide is an H chain consisting of the amino acid sequence of SEQ ID NO: 4, and the third polypeptide and the fourth polypeptide are a commonly shared L chain of SEQ ID NO: 9; (b) a bispecific antibody (Q1-G4k/J321-G4h/L45-k), wherein the first polypeptide is an H chain consisting of the amino acid sequence of SEQ ID NO: 1, the second polypeptide is an H chain consisting of the amino acid sequence of SEQ ID NO: 5, and the third polypeptide and the fourth polypeptide are a commonly shared L chain of SEQ ID NO: 9; (c) a bispecific antibody (Q31-z7/J326-z107/L2-k), wherein the first polypeptide is an H chain consisting of the amino acid sequence of SEQ ID NO: 2, the second polypeptide is an H chain consisting of the amino acid sequence of SEQ ID NO: 6, and the third polypeptide and the fourth polypeptide are a commonly shared L chain of SEQ ID NO: 8; (d) a bispecific antibody (Q64-z55/J344-z107/L45-k), wherein the first polypeptide is an H chain consisting of the amino acid sequence of SEQ ID NO: 3, the second polypeptide is an H chain consisting of the amino acid sequence of SEQ ID NO: 7, and the third polypeptide and the fourth polypeptide are a commonly shared L chain of SEQ ID NO: 9; (e) a bispecific antibody (Q64-z7/J326-z107/L334-k), wherein the first polypeptide is an H chain consisting of the amino acid sequence of SEQ ID NO: 10, the second polypeptide is an H chain consisting of the amino acid sequence of SEQ ID NO: 6, and the third polypeptide and the fourth polypeptide are a commonly shared L chain of SEQ ID NO: 30; (f) a bispecific antibody (Q64-z7/J344-z107/L406-k), wherein the first polypeptide is an H chain consisting of the amino acid sequence of SEQ ID NO: 10, the second polypeptide is an H chain consisting of the amino acid sequence of SEQ ID NO: 7, and the third polypeptide and the fourth polypeptide are a commonly shared L chain of SEQ ID NO: 33; (g) a bispecific antibody (Q85-G4k/J268-G4h/L406-k), wherein the first polypeptide is an H chain consisting of the amino acid sequence of SEQ ID NO: 11, the second polypeptide is an H chain consisting of the amino acid sequence of SEQ ID NO: 4, and the third polypeptide and the fourth polypeptide are a commonly shared L chain of SEQ ID NO: 33; (h) a bispecific antibody (Q85-G4k/J321-G4h/L334-k), wherein the first polypeptide is an H chain consisting of the amino acid sequence of SEQ ID NO: 11, the second polypeptide is an H chain consisting of the amino acid sequence of SEQ ID NO: 5, and the third polypeptide and the fourth polypeptide are a commonly shared L chain of SEQ ID NO: 30; (i) a bispecific antibody (Q153-G4k/J232-G4h/L406-k), wherein the first polypeptide is an H chain consisting of the amino acid sequence of SEQ ID NO: 12, the second polypeptide is an H chain consisting of the amino acid sequence of SEQ ID NO: 21, and the third polypeptide and the fourth polypeptide are a commonly shared L chain of SEQ ID NO: 33; (j) a bispecific antibody (Q354-z106/J259-z107/L324-k), wherein the first polypeptide is an H chain consisting of the amino acid sequence of SEQ ID NO: 13, the second polypeptide is an H chain consisting of the amino acid sequence of SEQ ID NO: 22, and the third polypeptide and the fourth polypeptide are a commonly shared L chain of SEQ ID NO: 29; (k) a bispecific antibody (Q360-G4k/J232-G4h/L406-k), wherein the first polypeptide is an H chain consisting of the amino acid sequence of SEQ ID NO: 14, the second polypeptide is an H chain consisting of the amino acid sequence of SEQ ID NO: 21, and the third polypeptide and the fourth polypeptide are a commonly shared L chain of SEQ ID NO: 33; (l) a bispecific antibody (Q360-z118/J300-z107/L334-k), wherein the first polypeptide is an H chain consisting of the amino acid sequence of SEQ ID NO: 15, the second polypeptide is an H chain consisting of the amino acid sequence of SEQ ID NO: 23, and the third polypeptide and the fourth polypeptide are a commonly shared L chain of SEQ ID NO: 30; (m) a bispecific antibody (Q405-G4k/J232-G4h/L248-k), wherein the first polypeptide is an H chain consisting of the amino acid sequence of SEQ ID NO: 16, the second polypeptide is an H chain consisting of the amino acid sequence of SEQ ID NO: 21, and the third polypeptide and the fourth polypeptide are a commonly shared L chain of SEQ ID NO: 28; (n) a bispecific antibody (Q458-z106/J346-z107/L408-k), wherein the first polypeptide is an H chain consisting of the amino acid sequence of SEQ ID NO: 17, the second polypeptide is an H chain consisting of the amino acid sequence of SEQ ID NO: 27, and the third polypeptide and the fourth polypeptide are a commonly shared L chain of SEQ ID NO: 34; (o) a bispecific antibody (Q460-z121/J327-z119/L334-k), wherein the first polypeptide is an H chain consisting of the amino acid sequence of SEQ ID NO: 18, the second polypeptide is an H chain consisting of the amino acid sequence of SEQ ID NO: 25, and the third polypeptide and the fourth polypeptide are a commonly shared L chain of SEQ ID NO: 30; (p) a bispecific antibody (Q499-z118/J327-z107/L334-k), wherein the first polypeptide is an H chain consisting of the amino acid sequence of SEQ ID NO: 19, the second polypeptide is an H chain consisting of the amino acid sequence of SEQ ID NO: 24, and the third polypeptide and the fourth polypeptide are a commonly shared L chain of SEQ ID NO: 30; (q) a bispecific antibody (Q499-z118/J327-z107/L377-k), wherein the first polypeptide is an H chain consisting of the amino acid sequence of SEQ ID NO: 19, the second polypeptide is an H chain consisting of the amino acid sequence of SEQ ID NO: 24, and the third polypeptide and the fourth polypeptide are a commonly shared L chain of SEQ ID NO: 31; (r) a bispecific antibody (Q499-z118/J346-z107/L248-k), wherein the first polypeptide is an H chain consisting of the amino acid sequence of SEQ ID NO: 19, the second polypeptide is an H chain consisting of the amino acid sequence of SEQ ID NO: 27, and the third polypeptide and the fourth polypeptide are a commonly shared L chain of SEQ ID NO: 28; (s) a bispecific antibody (Q499-z121/J327-z119/L404-k), wherein the first polypeptide is an H chain consisting of the amino acid sequence of SEQ ID NO: 20, the second polypeptide is an H chain consisting of the amino acid sequence of SEQ ID NO: 25, and the third polypeptide and the fourth polypeptide are a commonly shared L chain of SEQ ID NO: 32; (t) a bispecific antibody (Q499-z121/J339-z119/L377-k), wherein the first polypeptide is an H chain consisting of the amino acid sequence of SEQ ID NO: 20, the second polypeptide is an H chain consisting of the amino acid sequence of SEQ ID NO: 26, and the third polypeptide and the fourth polypeptide are a commonly shared L chain of SEQ ID NO: 31; and (u) a bispecific antibody (Q153-G4k/J142-G4h/L180-k), wherein the first polypeptide is an H chain consisting of the amino acid sequence of SEQ ID NO: 12, the second polypeptide is an H chain consisting of the amino acid sequence of SEQ ID NO: 170, and the third polypeptide and the fourth polypeptide are a commonly shared L chain of SEQ ID NO: 171.

One embodiment of the invention is the combination, antibody or use described above, wherein the FIX is administered in an amount of 10 U - 200 U FIX /kg body weight in a patient with hemophilia A treated with a multispecific antibody (that functionally substitutes for blood coagulation factor VIII) which comprises a first antigen- binding site that binds to coagulation factor IX and/or activated coagulation factor IX and a second antigen- binding site that binds to coagulation factor X. One embodiment of the invention is the combination, antibody or use described above, wherein additionally the FX is administered in an amount of 10 U - 200 U FX / kg body weight. One embodiment of the invention is the combination, antibody or use described above, wherein additionally the FII is administered in an amount of 10 U - 200 U FII / kg body weight. One embodiment of the invention is the combination, antibody or use described above, wherein additionally the FII and FX are administered in an amount of 10 U - 200 U FII / kg body weight and 10 U - 200 U FX / kg body weight. One embodiment of the invention is the combination, antibody or use described above, wherein prothrombin complex (PCC) is administered in amount of 10 U - 200 U PCC / kg body weight in a patient with hemophilia A treated with a multispecific antibody (that functionally substitutes for blood coagulation factor VIII) which comprises a first antigen- binding site that binds to coagulation factor IX and/or activated coagulation factor IX and a second antigen- binding site that binds to coagulation factor X.

Multispecific antibodies and antigen-binding molecules described herein comprise a first antigen-binding site and a second antigen-binding site that can specifically bind to at least two different types of antigens. While the first antigen-binding site and the second antigen-binding site are not particularly limited as long as they have an activity to bind to FIX and/or FIXa, and FX, respectively, examples include sites necessary for binding with antigens, such as antibodies, scaffold molecules (antibody-like molecules) or peptides, or fragments containing such sites. Scaffold molecules are molecules that exhibit function by binding to target molecules, and any polypeptide may be used as long as they are conformationally stable polypeptides that can bind to at least one target antigen. Examples of such polypeptides include antibody variable regions, fibronectin (WO 2002/032925), protein A domain (WO 1995/001937), LDL receptor A domain (WO 2004/044011, WO 2005/040229), ankyrin (WO 2002/020565), and such, and also molecules described in documents by Nygren et al. (Current Opinion in Structural Biology, 7: 463-469 (1997); and Journal of Immunol Methods, 290: 3-28 (2004)), Binz et al. (Nature Biotech 23: 1257-1266 (2005)), and Hosse et al. (Protein Science 15: 14-27(2006)). Furthermore, as mentioned in Curr Opin Mol Ther. 2010 Aug; 12(4): 487-95 and Drugs. 2008; 68(7): 901-12, peptide molecules that can bind to target antigens may be used.

Herein, multispecific antigen-binding molecules are not particularly limited as long as they are molecules that can bind to at least two different types of antigens, but examples include polypeptides containing the above-mentioned antigen-binding sites, such as antibodies and scaffold molecules as well as their fragments, and aptamers comprising nucleic acid molecules and peptides, and they may be single molecules or multimers thereof. Preferred multispecific antigen-binding molecules include multispecific antibodies that can bind specifically to at least two different antigens. Particularly preferred examples of antibodies which have an activity of functionally substituting for FVIII of the present invention include bispecific antibodies (BsAb) that can bind specifically to two different antigens (they may also be called dual specific antibodies).

In the present invention, the term "commonly shared L chain" refers to an L chain that can link with two or more different H chains, and show binding ability to each antigen. Herein, the term "different H chain(s)" preferably refers to H chains of antibodies against different antigens, but is not limited thereto, and also refers to H chains whose amino acid sequences are different from each other. Commonly shared L chain can be obtained, for example, according to the method described in WO 2006/109592.

The multispecific antigen-binding molecules of the present invention (preferably bispecific antibodies) are antibodies having specificity to two or more different antigens, or molecules comprising fragments of such antibodies. The antibodies of the present invention are not particularly limited, but are preferably monoclonal antibodies. Monoclonal antibodies used in the present invention include not only monoclonal antibodies derived from animals such as humans, mice, rats, hamsters, rabbits, sheep, camels, and monkeys, but also include artificially modified gene recombinant antibodies such as chimeric antibodies, humanized antibodies, and bispecific antibodies.

Furthermore, the L chains of an antibody which will become a multispecific antigen-binding molecule of the present invention may be different, but preferably have commonly shared L chains.

Multispecific antigen-binding molecules of the present invention are preferably recombinant antibodies produced using genetic recombination techniques (See, for example, Borrebaeck CAK and Larrick JW, THERAPEUTIC MONOCLONAL ANTIBODIES, Published in the United Kingdom by MACMILLAN PUBLISHERS LTD, 1990). Recombinant antibodies, can be obtained by cloning DNAs encoding antibodies from hybridomas or antibody-producing cells, such as sensitized lymphocytes, that produce antibodies, inserting them into suitable vectors, and then introducing them into hosts (host cells) to produce the antibodies.

Furthermore, antibodies of the present invention may include not only whole antibodies but also antibody fragments and low-molecular-weight antibodies (minibodies), and modified antibodies.

For example, antibody fragments or minibodies include diabodies (Dbs), linear antibodies, and single chain antibody (hereinafter, also denoted as scFvs) molecules. Herein, an "Fv" fragment is defined as the smallest antibody fragment that comprises a complete antigen recognition site and binding site.

An "Fv" fragment is a dimer (VH-VL dimer) in which an H chain variable region (VH) and an L chain variable region (VL) are strongly linked by non-covalent binding. The three complementarity determining regions (CDRs) of each of the variable regions interact with each other to form an antigen-binding site on the surface of the VH-VL dimer. Six CDRs confer the antigen-binding site to an antibody. However, one variable region (or half of the Fv comprising only three CDRs specific to an antigen) alone can recognize and bind to an antigen, though its affinity is lower than that of the entire binding site.

An Fab fragment (also called F(ab)) further comprises an L chain constant region and an H chain constant region (CH1). An Fab' fragment differs from an Fab fragment in that it additionally comprises several residues derived from the carboxyl terminus of the H chain CH1 region, comprising one or more cysteines from the hinge region of the antibody. Fab'-SH refers to an Fab' in which one or more cysteine residues of its constant region comprise a free thiol group. An F(ab') fragment is produced by cleavage of disulfide bonds between the cysteine residues in the hinge region of F(ab') 2 pepsin digest. Other chemically bound antibody fragments are also known to those skilled in the art.

Diabodies are bivalent minibodies constructed by gene fusion (Holliger, P. et al., Proc. Natl. Acad. Sci. USA 90: 6444-6448 (1993); EP 404,097; WO 93/11161).

Diabodies are dimers consisting of two polypeptide chains, in which each polypeptide chain comprises an L chain variable region (VL) and an H chain variable region (VH) linked with a linker short enough to prevent association of these two domains within the same chain, for example, a linker of preferably 2 to 12 amino acids, more preferably 3 to 10 amino acids, particularly about 5 amino acids. The polypeptide chain form a dimer since the linker between the VL and VH encoded on the same polypeptide is too short to form a single chain variable region fragment. Therefore, diabodies comprise two antigen-binding sites.

A single-chain antibody or an scFv antibody fragment comprises the VH and VL regions of an antibody, and these regions exist in a single polypeptide chain. In general, an Fv polypeptide further comprises a polypeptide linker between the VH and VL regions, and this enables an scFv to form a structure necessary for antigen binding (for a review on scFvs, see Pluckthun "The Pharmacology of Monoclonal Antibodies" Vol. 113 (Rosenburg and Moore ed. (Springer Verlag, New York) pp.269-315, 1994). In the context of the present invention, linkers are not particularly limited so long as they do not inhibit the expression of the antibody variable regions linked at their ends.

IgG-type bispecific antibodies can be secreted from hybrid hybridomas (quadromas) produced by fusing two kinds of hybridomas that produce IgG antibodies (Milstein C et al. Nature 1983, 305: 537-540). They can also be secreted by taking the L chain and H chain genes constituting the two kinds of IgGs of interest, a total of four kinds of genes, and introducing them into cells to coexpress the genes.

In this case, by introducing suitable amino acid substitutions to the CH3 regions of the H chains, IgGs having a heterogeneous combination of H chains can be preferentially secreted (Ridgway JB et al. Protein Engineering 1996, 9: 617-621; Merchant AM et al. Nature Biotechnology 1998, 16: 677-681; WO 2006/106905; Davis JH et al. Protein Eng Des Sel. 2010, 4: 195-202).

Regarding the L chains, since diversity of L chain variable regions is lower than that of H chain variable regions, commonly shared L chains that can confer binding ability to both H chains may be obtained. The antibodies of the present invention comprise commonly shared L chains. Bispecific IgGs can be efficiently expressed by introducing the genes of the commonly shared L chain and both H chains into cells.

Bispecific antibodies may be produced by chemically crosslinking Fab's. Bispecific F(ab') 2 can be produced, for example, by preparing Fab' from an antibody, using it to produce a maleimidized Fab' with ortho-phenylenedi-maleimide (o-PDM), and then reacting this with Fab' prepared from another antibody to crosslink Fab's derived from different antibodies (Keler T et al. Cancer Research 1997, 57: 4008-4014). The method of chemically linking an Fab'-thionitrobenzoic acid (TNB) derivative and an antibody fragment such as Fab'-thiol (SH) is also known (Brennan M et al. Science 1985, 229: 81-83).

Instead of a chemical crosslink, a leucine zipper derived from Fos and Jun may also be used. Preferential formation of heterodimers by Fos and Jun is utilized, even though they also form homodimers. Fab' to which Fos leucine zipper is added, and another Fab' to which Jun leucine zipper is added are expressed and prepared. Monomeric Fab'-Fos and Fab'-Jun reduced under mild conditions are mixed and reacted to form bispecific F(ab') 2 (Kostelny SA et al. J. of Immunology, 1992, 148: 1547-53). This method can be applied not only to Fab's but also to scFvs, Fvs, and such.

Furthermore, bispecific antibodies including sc(Fv) 2 such as IgG-scFv (Protein Eng Des Sel. 2010 Apr; 23(4): 221-8) and BiTE (Drug Discov Today 2005 Sep 15; 10(18): 1237-44.), DVD-Ig (Nat Biotechnol. 2007 Nov; 25(11): 1290-7. Epub 2007 Oct 14.; and MAbs. 2009 Jul; 1(4): 339-47. Epub 2009 Jul 10.), and also others (IDrugs 2010, 13: 698-700) including two-in-one antibodies (Science. 2009 Mar 20; 323(5921): 1610-4; and Immunotherapy 2009 Sep; 1(5): 749-51.), Tri-Fab, tandem scFv, and diabodies are known (MAbs. 2009 November; 1(6): 539-547). In addition, even when using molecular forms such as scFv-Fc and scaffold-Fc, bispecific antibodies can be produced efficiently by preferentially secreting a heterologous combination of Fcs (Ridgway JB et al., Protein Engineering 1996, 9: 617-621; Merchant AM et al. Nature Biotechnology 1998, 16: 677-681; WO 2006/106905; and Davis JH et al., Protein Eng Des Sel. 2010, 4: 195-202.).

A bispecific antibody may also be produced using a diabody. A bispecific diabody is a heterodimer of two cross-over scFv fragments. More specifically, it is produced by forming a heterodimer using VH(A)-VL(B) and VH(B)-VL(A) prepared by linking VHs and VLs derived from two kinds of antibodies, A and B, using a relatively short linker of about 5 residues (Holliger P et al. Proc Natl. Acad. Sci. USA 1993, 90: 6444-6448).

The desired structure can be achieved by linking the two scFvs with a flexible and relatively long linker comprising about 15 residues (single chain diabody: Kipriyanov SM et al. J. of Molecular Biology. 1999, 293: 41-56), and conducting appropriate amino acid substitutions (knobs-into-holes: Zhu Z et al. Protein Science. 1997, 6: 781-788; VH/VL interface engineering: Igawa T et al. Protein Eng Des Sel. 2010, 8: 667-77).

An sc(Fv) 2 that can be produced by linking two types ofscFvs with a flexible and relatively long linker, comprising about 15 residues, may also be a bispecific antibody (Mallender WD et al. J. of Biological Chemistry, 1994, 269: 199-206).

Examples of modified antibodies include antibodies linked to various molecules such as polyethylene glycol (PEG). The antibodies of the present invention include such modified antibodies. In the context of the present invention, the substance to which the modified antibodies are linked is not limited. Such modified antibodies can be obtained by chemically modifying obtained antibodies. Such methods are well established in the art.

The antibodies of the present invention include human antibodies, mouse antibodies, rat antibodies, or such, and their origins are not limited. They may also be genetically modified antibodies, such as chimeric or humanized antibodies.

Methods for obtaining human antibodies are known in the art. For example, transgenic animals carrying the entire repertoire of human antibody genes can be immunized with desired antigens to obtain desired human antibodies (see International Patent Application WO 93/12227, WO 92/03918, WO 94/02602, WO 94/25585, WO 96/34096, and WO 96/33735).

Genetically modified antibodies can also be produced using known methods. Specifically, for example, chimeric antibodies may comprise H chain and L chain variable regions of an immunized animal antibody, and H chain and L chain constant regions of a human antibody Chimeric antibodies can be obtained by linking DNAs encoding the variable regions of the antibody derived from the immunized animal, with DNAs encoding the constant regions of a human antibody, inserting this into an expression vector, and then introducing it into host cells to produce the antibodies.

Humanized antibodies are modified antibodies often referred to as "reshaped" human antibodies. A humanized antibody is constructed by transferring the CDRs of an antibody, derived from an immunized animal to the complementarity determining regions of a human antibody. Conventional genetic recombination techniques for such purposes are known (see European Patent Application Publication No. EP 239400; International Publication No. WO 96/02576; Sato K et al., Cancer Research 1993, 53: 851-856; International Publication No. WO 99/51743).

The multispecific antigen-binding molecules of the present invention are those that recognize FIX and/or FIXa, and FX, and functionally substitute for cofactor function of FVIII, and characterized in that the molecules have a higher FXa generation-promoting activity compared to hA69-KQ/hB26-PF/hAL-AQ (described in WO 2006/109592) which is known as a bispecific antibody that functionally substitutes for FVIII. Furthermore, antibodies of the present invention usually have a structure which comprises a variable region of an anti-FIXa antibody and a variable region of an anti-FX antibody.

A multispecific antigen-binding molecule of the present invention functionally substitutes for FVIII, which comprises a first antigen-binding site that recognizes FIX and/or FIXa and a second antigen-binding site that recognizes FX, wherein the function that substitutes for the function of FVIII is caused by a higher FXa generation-promoting activity compared to the activity of the bispecific antibody (hA69-KQ/hB26-PF/hAL-AQ) which comprises H chains consisting of SEQ ID NOs: 165 and 166, and a commonly shared L chain consisting of SEQ ID NO: 167.

A multispecific antigen-binding molecule of the present invention comprises a first polypeptide and a third polypeptide comprising an antigen-binding site that recognizes FIX and/or FIXa, and a second polypeptide and a fourth polypeptide comprising an antigen-binding site that recognizes FX. The first polypeptide and the third polypeptide, and the second polypeptide and the fourth polypeptide each include the antigen-binding site of the antibody H chain and the antigen-binding site of the antibody L chain.

For example, in a multispecific antigen-binding molecule of the present invention, the first polypeptide and the third polypeptide include an antigen-binding site of an H chain and L chain of an antibody against FIX or FIXa, respectively; and the second polypeptide and the fourth polypeptide comprise an antigen-binding site of an H chain and L chain of an antibody against FX, respectively.

At this time, the antigen-binding sites of the antibody L chain included in the first polypeptide and the third polypeptide, and the second polypeptide and the fourth polypeptide may be commonly shared L chains.

A polypeptide comprising an antigen-binding site of an antibody L chain in the present invention is preferably a polypeptide which comprises all or a part of the sequence of the antibody L chain which binds to FIX, FIXa and/or FX.

In the present invention, the phrase "functionally substitute for FVIII" means that FIX and/or FIXa, and FX is recognized, and activation of FX is promoted (FXa generation is promoted).

In the present invention, "FXa generation-promoting activity" can be confirmed by evaluating the multispecific antigen-binding molecules of the present invention using, for example, a measurement system comprising FXIa (FIX activating enzyme), FIX, FX, F synthetic substrate S-2222 (synthetic substrate of FXa), and phospholipids. This measurement system shows the correlation between the severity of the disease and clinical symptoms in hemophilia A cases (Rosen S, Andersson M, Blomback M et al. Clinical applications of a chromogenic substrate method for determination of FVIII activity. Thromb Haemost 1985, 54: 811-23). That is, in the present measurement system, test substances that show higher FXa generation-promoting activity are expected to show better hemostatic effects against bleeding episodes in hemophilia A. With these results, if a multispecific antigen-binding molecule having activity of functionally substituting for FVIII is a molecule having a higher activity than hA69-KQ/hB26-PF/hAL-AQ, it may yield excellent blood coagulation-promoting activity, and excellent effects may be obtained as a pharmaceutical component for preventing and/or treating bleeding, a disease accompanying bleeding, or a disease caused by bleeding. To obtain excellent effects as the above-mentioned pharmaceutical component, for example, FXa generation-promoting activity measured under the conditions described in Example 2 of US 2013/0330345 is preferably not less than that of hA69-KQ/hB26-PF/hAL-AQ, and in particular, the activity is more preferably the same as or not less than that of Q153-G4k/J142-G4h/L180-k. Herein, the "FXa generation-promoting activity" is the value obtained by subtracting the change in absorbance upon 20 minutes in a solvent from the change in absorbance upon 20 minutes in an antibody solution.

A preferred embodiment of the present invention is a multispecific antibody that functionally substitutes for FVIII, which recognizes FIX and/or FIXa, and FX.

The above-mentioned multispecific antibodies of the present invention are preferably antibodies which comprise H chain CDRs of anti-FIX/FIXa antibodies or CDRs functionally equivalent to them, and H chain CDRs of anti-FX antibodies or CDRs functionally equivalent to them.

In one aspect of the present invention, in the combination of the multispecific antibody with FIX a prothrombin complex concentrates containing FIX, FII, FVII and FX (4-component PCC), or prothrombin complex concentrates containing only or mainly FIX, FII and FX (3component PCC) can be used. The 3-component PCCs is one preferred embodiment for the combination of the present invention due to the lack of FVII, which might form a competition for its active form FVIIa. In another aspect of the present invention. In addition to FIX in the combination with the multispecific antibody, FII, FX; or FII and FX can be used as prepared preparation mixtures. In addition to the aforementioned proteins also the structural protein fibrinogen, or a antifibrinolytic drug such as tranexamic acid or aprotinin could be added.

Blood coagulation factors maybe exist in their inactive precursor forms as zymogen ( e.g. FIX) or as activated forms ( e.g. FIXa). A zymogen requires a biochemical change (such as a hydrolysis reaction revealing the active site, or changing the configuration to reveal the active site) for it to become an active enzyme. The biochemical change usually occurs in a lysosome where a specific part of the precursor enzyme is cleaved in order to activate it. The activated blood coagulation factors are typically abbreviated as e.g FIXa, FXa etc. The activation mechanism of e.g. of coagulation factor IX is described in Biol Chem. 2009 May-Jun;390(5-6):391-400.

The terms "blood coagulation factor", "coagulation factor", or "(blood) coagulation factor" or in abbreviated form only "F" before the respective blood coagulation factor number (e.g. FVIII, FIX, FX etc) as used herein are interchangeable and refer to respective human blood coagulation factors of the human coagulation system. In their activated form they are abbreviated e.g. as FVIIIa, FIXa, FXa. In their no-activated form they are abbreviated as FVIII, FIX, FX etc..

"Coagulation factor IX" (FIX) is a zymogen, an inactive precursor. It is processed to remove the signal peptide, and then cleaved by factor XIa (of the contact pathway) or factor VIIa (of the tissue factor pathway) to produce a two-chain form where the chains are linked by a disulfide bridge (Di Scipio RG, et al, J. Clin. Invest. 61 (1978) 1528-38; Taran LD Biochemistry Mosc. 62 (1997) 685-93). When activated into factor IXa, in the presence of Ca²⁺, membrane phospholipids, and a Factor VIII cofactor, it hydrolyses one arginine-isoleucine bond in Factor X to form factor Xa. Therefore the term "Coagulation factor IX" ("FIX") as used herein refers to the non-activated coagulation factor IX.

Deficiency of factor IX causes Christmas disease (hemophilia B) (Biggs, R; et al British Medical Journal 2 (4799) 1952 1378-82). Over 100 mutations of factor IX have been described; some cause no symptoms, but many lead to a significant bleeding disorder. The original Christmas disease mutation was identified by sequencing of Christmas' DNA, revealing a mutation which changed a cysteine to a serine (Taylor, S. A.; et al, Thrombosis and haemostasis 67 (1992) 63-65. Recombinant factor IX is used to treat Christmas disease, and is commercially available as "BeneFIX (R)" "Alprolix(R)", and "Rixubis(R)" ( all brand names for a recombinant Factor IX products). Some rare mutations of factor IX result in elevated clotting activity, and can result in clotting diseases, such as deepvein thrombosis (Simioni P, et al, N. Engl. J. Med. 361 (2009) 1671-5).

FIX is synthesized as a single polypeptide chain 415 amino acids in length. FIX is present in blood as an inactive precursor molecule that consists of (1) a gamma-carboxyglutamic acid containing domain ("Gla domain"), (2) and (3) two epidermal growth factor-like domains ("EGF-1 domain", "EGF-2 domain"), (4) an activation peptide region ("AP region"), and (5) a serine protease domain. FIX undergoes extensive post-translational modification during transit through the endoplasmatic reticulum and Golgi apparatus: removal of the signal sequence; gamma-carboxylation of twelve Glu residues in the Gla domain by vitamin K dependent gamma-glutamyl carboxylase, a hepatic microsomal enzyme; N-glycosylation of N-157 and N-167 in the AP region; O-glycosylation of S-53 and S-61 in the Gla domain and T-159, T-169, T-172 and T-179 in the AP region; beta-hydroxylation at Asp-64 in the EGF-1 domain; sulfation of Tyr-155 and phosphorylation of Ser-158, both in the AP region.

In Haemophilia B, the deficiency is either in the amount or in the function of FIX. This disease is successfully treated by replacement therapy consisting of the administration of preparations of human plasma derived (pdFIX) or recombinant coagulation factor IX (rFIX). Plasma derived products are either prothrombin complex concentrates (which have been used in the past for the treatment of Haemophilia B) or purified FIX concentrates (mainly affinity purified factor IX). rFIX has been extensively characterised with respect to post-translational modifications. Despite minor differences to the pdFIX, specific activities and pharmacological effectiveness are comparable.

Biochemical comparison between pdFIX and CHO derived rFIX showed an indistinguishable secondary/tertiary structure as measured by fluorescence, circular dichroism or analytical ultracentrifugation. Minor differences were detected in post-translational modifications. Whereas in pdFIX all 12 Glu residues in the Gla domain are occupied (i.e. transformed to Gla), only 10 of the 12 sites are fully occupied in rFIX ("undercarboxylation" of Gla-40 or Gla-40 and Gla-36, respectively). N-linked glycans are fully sialylated and show high heterogeneity in pdFIX (however, this may also be due to the fact that pdFIX is prepared from plasma pools having diverse plasma donations); low hetereogeneity and often incomplete sialysation in rFIX. Ser-53 is Xyl-Xyl-Gic-glycosylated in rFIX whereas in pdFIX Ser-53 contains additional Xyl-Glc- glycosylation (Ser-61 contains NeuAc-Gal-GlcNAc-Fuc-in both forms). rFIX from CHO cells exhibits glycosylation with carbohydrates capped with sialic acid alpha(2-3)-galactose groups (CHO cells lack alpha(2-6)-sialyltransferase) whereas pdFIX contains terminal sialic acid alpha(2-6)-galactose moieties. Human host cells for expressing rFIX (such as HEK 293 cells) contain alpha(2-3)- and alpha(2-6)-sialyltransferases; accordingly HEK 293 derived rFIX differs in this respect from commercial CHO-derived rFIX (White et al., Thromb.Haemost. 78(1) (1997), 261-265; Bond et al., Sem.Hematol. 35 (2) (1998), Suppl.2, 11-17; Bebgie et al., Thromb.Haemost.94 (2005), 1138-1147).

It has been speculated whether a lower degree of phosphorylation of Ser-155 in the AP region and the lower degree of sulfation of Tyr-158 are responsible for the lower in-vivo recovery of rFIX (37.81 +- 14.0 % of rFIX compared to 52.61 +- 12.36 for pdFIX purified with monoclonal antibodies (White et al. (1997)). Griffith et al. (J.Thromb.Haemost. 5 (2007), Suppl.2: P-M-043) reported that N-Glycan sialylation is important for in vivo recovery of rFIX. In WO 2007/101681 A1 rFIX products with improved in vivo recovery are provided comprising at least 25 % and less than 98 % of fully phosphorylated and sulfated rFIX.

Elimination half life of CHO expressed rFIX and immunopurified pdFIX are comparable (18.10 +- 5.10 hours and 17.66 +- 5.31 hours, respectively (White et al., 1997)). Based on a report that deletion of the AP region (a del(155-177) mutant showed a terminal catabolic half life increase of 45 % compared to the wild-type form (Bebgie et al. (2005)), Chang et al. (J.Thromb.Haemost. 5 (2007), Suppl.2: O-M-088) treated FIX with neuraminidase and N- and O-glycanase to remove both, the N- and O-linked carbohydrates. De-glycosylated FIX had a significantly lower recovery than untreated FIX, whereas recovery of the de-glycosylated form were not statistically different in rFIX and pdFIX. It was therefore concluded that this suggested that glycosylation plays a major role in determining the recovery of FIX. It was further concluded that the role of sulfation/phosphorylation play a "relatively minor" role in in vivo recovery. Half life or activity data were not reported for the de-glycosylated forms of rFIX and pdFIX in Chang et al..

In clinical studies, rFIX has been shown to be safe and effective, but a 20 to 50 % higher dosage than for pdFLX is needed for successful treatment. This is due to a 30 to 50 % lower in vivo recovery for CHO derived rFIX than for pdFIX (as described above), as also revealed by pharmacokinetic data collected from pre-clinical and clinical studies, where pdFIX and rFIX are compared in different animal models, and clinical studies in haemophilia B patients. However, the circulating half-life of rFIX is not distinguishable from pdFIX preparations.

There had been various attempts to improve FIX drugs, e.g. (for rFIX) increased mRNA production, reduced binding to collagen IV, increasing the specific activity and improving the recovery by making rFIX more similar to pdFIX (Pipe, Sem.Thromb.Hemost. 30 (2) (2004), 227-237; WO 2007/101681 A1); (for pdFIX) enrichment and specific purification (US 5,639,857 A). However, there is still a strong need for improved FIX preparations which can be administered in a lower dosage or in larger time intervals than conventional FIX preparations for a successful treatment. Whereas most strategies in the prior art concentrate on improving recovery and increasing FIX activity, strategies which aim at prolongation of half life of the protein are rare, mainly because half life of rFIX and pdFIX are the same. This is mainly due to the known sensibility of the FIX protein against (even minor) chemical modification or mutations and the potential immunological effects of introducing mutations into a human protein (Bebgie et al. (2005); Kaufman, Thromb.Haemost. 79 (1998), 1068-1079; Hansson et al., J Thromb.Haemost. 3 (2005), 2633-2648; Wojcik et al., Biochem.J. 323 (1997), 629-636)

The term "coagulation factor IX" (FIX) as used herein shall be any form of factor IX molecule with the typical characteristics of blood coagulation factor IX. FIX shall include FIX from plasma (pdFIX) and any form of rFIX which is capable of curing bleeding disorders in a patient which are caused by deficiencies in FIX (e.g. haemophilia B). FIX is comprised of the Gla domain, two EGF domains (EGF-1 and EGF-2), an AP region and a serine protease domain. FIX according to the present inventionshall have the same amino acid sequence as human pdFIX and human rFIX and all functional variations thereof, i.e. variations (both, in amino acid sequence and post-translational modifications) which provide a comparable or improved in vivo activity of FIX. For curing the respective FIX related bleeding disorders in animals, the corresponding FIX sequences may be applied or those FIX forms which show sufficient cross-activity in related animal species. Furthermore, FIX according to the present invention shows all post-translational modifications necessary for a proper functioning of the protein in vivo. Ample literature is available describing functional forms of FIX, for example a naturally occurring Ala/Thr exchange at position 148; suitable FIX molecules which can be covalently coupled to the water-soluble hydrophilic polymers according to the present invention are described e.g. in White et al. (1997); Pipe (2004); WO 2007/101681 A1; US 5,639,857 A; Bebgie et al. (2005); Kaufman (1998); Hansson et al. (2005); Wojcik et al. (1997). Preferably, the FIX according to the present invention is a recombinantly produced FIX. The term "recombinant" when used with reference to FIX indicates that FIX has been produced by the introduction of a heterologous or non-naturally occurring nucleic acid or protein into a host cell, or the alteration of a native nucleic acid or protein in a host cell. Thus, for example, recombinant cells express genes that are not found within the native (non-recombinant) form of the cell, or express wild type and variant genes that are not in the native position in the genome of the cell, or express native genes that are otherwise abnormally expressed, under expressed or not expressed at all. The term "biologically produced" FIX covers all FIX forms being produced by organisms or cells without further chemical modification (not performable by such organisms or cells) after FIX has been isolated from such organisms or cells.

Commercially available recombinant factor IX products include "BeneFIX (R)", "Alprolix(R) (recombinant Factor IX Fc fusion protein with elongated halflife)"-and "Rixubis(R)" (all brand names for a recombinant Factor IX product Benefix(TM)).

Commercially available recombinant factor IX products are often manufactured by using stable transfected Chinese hamster ovary (CHO) cells. CHO cells provide capacity for glycosylation and other post-translational modifications. With these cells, large-scale suspension cultures can be maintained without the addition of animal- or human-derived raw material. In the manufacture of one of these commercial products (marketed under the trade name Benefix(TM)) rFIX is co-expressed with the endopeptidase PACE/furin and is highly purified via multiple filtration and chromatographic steps.

The term "heterologous" when used with reference to portions of a nucleic acid indicates that the nucleic acid comprises two or more subsequences that are not found in the same relationship to each other in nature. In one example, this term refers to a nucleic acid that is not in its native position in the genome. In another example, the nucleic acid is recombinantly produced, having two or more sequences from unrelated genes arranged to make a new functional nucleic acid, e.g. a promoter from one source and a coding region from another source. Similarly, a heterologous protein indicates that the protein comprises two or more subsequences that are not found in the same relationship to each other in na-ture (e.g. a fusion protein), or that it is a protein derived from a heterologous nucleic acid.

Any biologically active derivative of FIX may be modified thereby including any derivative of FIX having qualitatively the same functional and/or biological properties of FIX such as binding properties, and/or the same structural basis, such as a peptidic backbone. Minor deletions, additions and/or substitutions of amino acids of the polypeptide sequence of FIX which are not abolishing the biological activity of said polypeptide (i.e. reducing the activity to below 10 % or even below 5 % of the wild type form (= 100 %)) are also included in the present application as biologically active derivatives, especially those with improved specific activity (above 100 % activity of the wild-type form). The FIX according to the present invention may be derived from any vertebrate, e.g. a mammal. In one specific example of the present invention, the FIX is human FIX. The FIX according to the present invention may be produced by any method known in the art. This may include any method known in the art for the production of recombinant DNA by genetic engineering, e.g. via reverse transcription of RNA and/or amplification of DNA. Additionally, the recombinant DNA coding for FIX, e.g. a plasmid, may also contain a DNA sequence encoding a selectable marker for selecting the cells which have been successfully transfected with the plasmid. In an example of the present invention, the plasmid may also confer resistance to a selectable marker, e.g. to the antibiotic drug G418, by delivering a resistance gene, e.g. the neo resistance gene conferring resistance to G418.

The production of rFIX may include any method known in the art for the introduction of recombinant DNA into eukaryotic cells by transfection, e.g. via electroporation or microinjection. For example, the recombinant expression of human FIX can be achieved by introducing an expression plasmid containing the human FIX encoding DNA sequence under the control of one or more regulating sequences such as a strong promoter, into a suitable host cell line by an appropriate transfection method resulting in cells having the introduced sequences stably integrated into the genome. The calcium-phosphate co-precipitation method is an example of a transfection method which may be used according to the present invention.

The term "amino acid" within the scope of the present invention is meant to include all naturally occurring L .alpha.-amino acids. The one and three letter abbreviations for naturally occurring amino acids are used herein (Lehninger, Biochemistry, 2d ed., Worth Publishers, New York, 1995: 71 -92).

The term"coagulation factor II" (FII, prothrombin) as used herein refers to any form of factor II molecule with the typical characteristics of blood coagulation factor II. Blood coagulation factor II is a zymogen also known as prothrombin and is proteolytically cleaved to form th activated blood coagulation factor II (FIIa) aslo known as thrombin in the coagulation cascade, which ultimately results in the reduction of blood loss. Thrombin in turn acts as a serine protease that converts soluble fibrinogen into insoluble strands of fibrin, as well as catalyzing many other coagulation-related reactions.

"Prothrombin complex concentrate" (PCC, trade names Beriplex (R), Octaplex (R), Kcentra(R) , Cofact(R) , among others) is a combination of blood coagulation factors II, VII, IX and X, as well as protein C and S, prepared from fresh-frozen human blood plasma. It is used to reverse the effects of oral anticoagulation therapy when bleeding occurs (e.g. in the brain or gut) requiring rapid action to accelerate coagulation. It is available as a powder and solvent for solution for injection.

"Coagulation factor X", also known by the eponym Stuart-Prower factor or as prothrombinase, thrombokinase or thromboplastin, is an enzyme of the coagulation cascade. The term "coagulation factor X" (FX) as used herein shall be any form of factor X molecule with the typical characteristics of blood coagulation factor X. Factor X is synthesized in the liver and requires vitamin K for its synthesis. Factor X is activated into factor Xa by both factor IX (with its cofactor, factor VIII in a complex known as intrinsic Xase) and factor VII with its cofactor, tissue factor (a complex known as extrinsic Xase). It acts by cleaving prothrombin in two places (an arg-thr and then an arg-ile bond), which yields the active thrombin. This process is optimized when factor Xa is complexed with activated co-factor V in the prothrombinase complex. Factor Xa is inactivated by protein Z-dependent protease inhibitor (ZPI), a serine protease inhibitor (serpin). The affinity of this protein for factor Xa is increased 1000-fold by the presence of protein Z, while it does not require protein Z for inactivation of factor XI. Defects in protein Z lead to increased factor Xa activity and a propensity for thrombosis. The half life of factor X is 40-45 hours. Factor X is part of fresh frozen plasma and Prothrombin complex and Prothrombin complex concentrates . A commercially available concentrate is Factor X P Behring' manufactured by CSL Behring. Bio Products Laboratory has a high purity Factor X currently in development.

Factor VII (blood-coagulation factor VII) is one of the proteins that causes blood to clot in the coagulation cascade. It is an enzyme of the serine protease class. The term "coagulation factor VII" (FVII) as used herein shall be any form of factor VII molecule with the typical characteristics of blood coagulation factor VII. A recombinant form of its activated form human factor VIIa (eptacog alfa [activated], NovoSeven) is approved for the treatment of uncontrolled bleeding in hemophilia patients. There have been safety concerns when used in severe uncontrollable bleeding.

The main role of factor VII (FVII) is to initiate the process of coagulation in conjunction with tissue factor (TF/coagulation factor III/ FIII). Tissue factor is found on the outside of blood vessels - normally not exposed to the bloodstream. Upon vessel injury, tissue factor is exposed to the blood and circulating factor VII. Once bound to TF, FVII is activated to FVIIa by different proteases, among which are thrombin (factor IIa), factor Xa, IXa, XIIa, and the FVIIa-TF complex itself. The complex of factor VIIa with TF catalyzes the conversion of factor IX and factor X into the active proteases, factor IXa and factor Xa, respectively (Wajima T, et al, Clin Pharmacol Ther 86 (2009). 290-8). The action of the factor is impeded by tissue factor pathway inhibitor (TFPI), which is released almost immediately after initiation of coagulation. Factor VII is vitamin K dependent; it is produced in the liver. Use of warfarin or similar anticoagulants decreases hepatic synthesis of FVII.

The terms "bind to", "recognize" , "specifically bind to" or "anti-" as used herein are interchangeable and refer to refer to mutlispecific antibody or its antigen binding site that is capable of binding the respective antigen with sufficient affinity such that the antibody is useful as a diagnostic and/or therapeutic agent in targeting. Preferably the multispecific antibody as described herein is bispecific and binds to FIX and/or FIXa (activated form of FIX) as the first antigen, and to FX as the second antigen, respectively In one embodiment, the extent of binding of an anti-Bsab FIX/FX, antibody to an unrelated, non-FIX, non-FIXa, non-FX protein is less than about 10% of the binding of the antibody to FIX, FIXa, FX, respectively, as measured, e.g., by a radioimmunoassay (RIA).

The term "antigen-binding site" as used herein denotes the region(s) of an antibody molecule to which a ligand actually binds. The term "antigen-binding site" include antibody heavy chain variable domains (VH) and/or an antibody light chain variable domains (VL), or pairs of VH/VL, and can be derived from whole antibodies or antibody fragments such as single chain Fv, a VH domain and/or a VL domain, Fab, or (Fab)2. In one embodiment of the current invention each of the antigen-binding sites comprises an antibody heavy chain variable domain (VH) and/or an antibody light chain variable domain (VL), and preferably is formed by a pair consisting of an antibody light chain variable domain (VL) and an antibody heavy chain variable domain (VH), wherein antibody light chain variable domain (VL) is preferably part of a commly shared L chain.

The term "wherein the treatment is in combination with a coagulation factor IX" refers to the combined treatment of the relevant disorder with a) a multispecific antibody which comprises a first antigen- binding site that binds to coagulation factor IX and/or activated coagulation factor IX and a second antigen- binding site that binds to coagulation factor X, and b) a coagulation factor IX. The combined treatment can be simultaneous or sequential wherein preferably there is a time period while both (or all) active agents simultaneously exert their biological activities. Said multispecific antibody and FIX are co-administered either simultaneously or sequentially (e.g. via an intravenous (i.v.) through a continuous infusion).

Multispecific antigen-binding molecules described herein comprise a first antigen-binding site and a second antigen-binding site that can specifically bind to at least two different types of antigens. While the first antigen-binding site and the second antigen-binding site are not particularly limited as long as they bind to FIX and/or FIXa, and FX, respectively, examples include sites necessary for binding with antigens, such as antibodies, scaffold molecules (antibody-like molecules) or peptides, or fragments containing such sites. Scaffold molecules are molecules that exhibit function by binding to target molecules, and any polypeptide may be used as long as they are conformationally stable polypeptides that can bind to at least one target antigen. Examples of such polypeptides include antibody variable regions, fibronectin (WO 2002/032925), protein A domain (WO 1995/001937), LDL receptor A domain (WO 2004/044011, WO 2005/040229), ankyrin (WO 2002/020565), and such, and also molecules described in documents by Nygren et al. (Current Opinion in Structural Biology, 7: 463-469 (1997); and Journal of Immunol Methods, 290: 3-28 (2004)), Binz et al. (Nature Biotech 23: 1257-1266 (2005)), and Hosse et al. (Protein Science 15: 14-27(2006)). Furthermore, as mentioned in Curr Opin Mol Ther. 2010 Aug; 12(4): 487-95 and Drugs. 2008; 68(7): 901-12, peptide molecules that can bind to target antigens may be used.

Herein, multispecific antigen-binding molecules are not particularly limited as long as they are molecules that can bind to at least two different types of antigens, but examples include polypeptides containing the above-mentioned antigen-binding sites, such as antibodies and scaffold molecules as well as their fragments, and aptamers comprising nucleic acid molecules and peptides, and they may be single molecules or multimers thereof. Preferred multispecific antigen-binding molecules include multispecific antibodies that can bind specifically to at least two different antigens. Particularly preferred examples of antibodies which have an activity of functionally substituting for FVIII of the present invention include bispecific antibodies (BsAb) that can bind specifically to two different antigens (they may also be called dual specific antibodies).

In the present invention, the term "commonly shared L chain" refers to an L chain (light chain) of an antibody that can link with two or more different H chains (heavy chains) of antibody, and show binding ability to each antigen. Herein, the term "different H chain(s)" preferably refers to H chains of antibodies against different antigens, but is not limited thereto, and also refers to H chains whose amino acid sequences are different from each other. Commonly shared L chain can be obtained, for example, according to the method described in WO 2006/109592.

The multispecific antigen-binding molecules of the present invention (preferably bispecific antibodies) are antibodies having specificity to two or more different antigens, or molecules comprising fragments of such antibodies. The antibodies of the present invention are not particularly limited, but are preferably monoclonal antibodies. Monoclonal antibodies used in the present invention include not only monoclonal antibodies derived from animals such as humans, mice, rats, hamsters, rabbits, sheep, camels, and monkeys, but also include artificially modified gene recombinant antibodies such as chimeric antibodies, humanized antibodies, and bispecific antibodies.

Furthermore, the L chains of an antibody which will become a multispecific antigen-binding molecule of the present invention may be different, but preferably have commonly shared L chains.

Multispecific antigen-binding molecules of the present invention are preferably recombinant antibodies produced using genetic recombination techniques (See, for example, Borrebaeck CAK and Larrick JW, THERAPEUTIC MONOCLONAL ANTIBODIES, Published in the United Kingdom by MACMILLAN PUBLISHERS LTD, 1990). Recombinant antibodies, can be obtained by cloning DNAs encoding antibodies from hybridomas or antibody-producing cells, such as sensitized lymphocytes, that produce antibodies, inserting them into suitable vectors, and then introducing them into hosts (host cells) to produce the antibodies.

Furthermore, antibodies of the present invention may include not only whole antibodies but also antibody fragments and low-molecular-weight antibodies (minibodies), and modified antibodies.

The multispecific antigen-binding molecules of the present invention are those that recognize FIX and/or FIXa, and FX, and functionally substitute for cofactor function of FVIII, and characterized in that the molecules have a higher FXa generation-promoting activity compared to hA69-KQ/hB26-PF/hAL-AQ (described in WO 2006/109592) which is known as a bispecific antibody that functionally substitutes for FVIII. Furthermore, antibodies of the present invention usually have a structure which comprises a variable region of an anti-FIXa antibody and a variable region of an anti-FX antibody.

More specifically, the present invention provides a multispecific antigen-binding molecule that functionally substitutes for FVIII, which comprises a first antigen-binding site that recognizes FIX and/or FIXa and a second antigen-binding site that recognizes FX, wherein the function that substitutes for the function of FVIII is caused by a higher FXa generation-promoting activity compared to the activity of the bispecific antibody (hA69-KQ/hB26-PF/hAL-AQ) which comprises H chains consisting of SEQ ID NOs: 165 and 166, and a commonly shared L chain consisting of SEQ ID NO: 167.

A multispecific antigen-binding molecule of the present invention comprises a first polypeptide and a third polypeptide comprising an antigen-binding site that recognizes FIX and/or FIXa, and a second polypeptide and a fourth polypeptide comprising an antigen-binding site that recognizes FX. The first polypeptide and the third polypeptide, and the second polypeptide and the fourth polypeptide each include the antigen-binding site of the antibody H chain and the antigen-binding site of the antibody L chain.

For example, in a multispecific antigen-binding molecule of the present invention, the first polypeptide and the third polypeptide include an antigen-binding site of an H chain and L chain of an antibody against FIX or FIXa, respectively; and the second polypeptide and the fourth polypeptide comprise an antigen-binding site of an H chain and L chain of an antibody against FX, respectively.

At this time, the antigen-binding sites of the antibody L chain included in the first polypeptide and the third polypeptide, and the second polypeptide and the fourth polypeptide may be commonly shared L chains.

A polypeptide comprising an antigen-binding site of an antibody L chain in the present invention is preferably a polypeptide which comprises all or a part of the sequence of the antibody L chain which binds to FIX, FIXa and/or FX.

In the present invention, the phrase "functionally substitute for FVIII" means that FIX and/or FIXa, and FX is recognized, and activation of FX is promoted (FXa generation is promoted).

In the present invention, "FXa generation-promoting activity" can be confirmed by evaluating the multispecific antigen-binding molecules of the present invention using, for example, a measurement system comprising FXIa (FIX activating enzyme), FIX, FX, F synthetic substrate S-2222 (synthetic substrate of FXa), and phospholipids. This measurement system shows the correlation between the severity of the disease and clinical symptoms in hemophilia A cases (Rosen S, Andersson M, Blomback M et al. Clinical applications of a chromogenic substrate method for determination of FVIII activity. Thromb Haemost 1985, 54: 811-23). That is, in the present measurement system, test substances that show higher FXa generation-promoting activity are expected to show better hemostatic effects against bleeding episodes in hemophilia A. With these results, if a multispecific antigen-binding molecule having activity of functionally substituting for FVIII is a molecule having a higher activity than hA69-KQ/hB26-PF/hAL-AQ, it may yield excellent blood coagulation-promoting activity, and excellent effects may be obtained as a pharmaceutical component for preventing and/or treating bleeding, a disease accompanying bleeding, or a disease caused by bleeding. To obtain excellent effects as the above-mentioned pharmaceutical component, for example, FXa generation-promoting activity measured under the conditions described in Example 2 of US 2013/0330345 is preferably not less than that of hA69-KQ/hB26-PF/hAL-AQ, and in particular, the activity is more preferably the same as or not less than that of Q153-G4k/J142-G4h/L180-k. Herein, the "FXa generation-promoting activity" is the value obtained by subtracting the change in absorbance upon 20 minutes in a solvent from the change in absorbance upon 20 minutes in an antibody solution ( see also US 2013/0330345).

In one embodiment of antibodies, combination or use of the present invention, since the antibodies used in the present invention functionally substitute for factor FVIII, they are expected to become effective pharmaceutical agents against diseases resulting from decrease in activity (function) of this cofactor. Examples of the above-mentioned diseases include bleeding, diseases accompanying bleeding, or a disease caused by bleeding. In particular, there may have excellent therapeutic effects on hemophilias, in which bleeding disorders are caused by a deficiency or decrease of FVIII/FVIIIa function. Among the hemophilias, they are expected to become excellent therapeutic agents for hemophilia A, in which bleeding disorders are caused by a hereditary deficiency or decrease of FVIII/FVIIIa function.

In the context of the present invention, bleeding, diseases accompanying bleeding, and/or diseases caused by bleeding preferably refer to diseases that develop and/or progress due to reduction or deficiency in activity of FVIII and/or activated coagulation factor VIII (F.VIIIa). Such diseases include the above-described hemophilia A, diseases in which an inhibitor against FVIII/FVIIIa appear, acquired hemophilia, von Willebrand's disease, and such, but are not particularly limited thereto.

The following examples and figures are provided to aid the understanding of the present invention, the true scope of which is set forth in the appended claims.

### Experimental procedures:

### Example 1

### Thrombin generation in FVIII deficient plasma

Plasma samples which were deficient in FVIII (FVIII deficient plasma, Siemens) , (however containing normal levels of non activated FIX, FX and FII) were used as a model of a deficiency or malfunction of coagulation factor VIII (especially as model a hemophilia A patient samples).

The bispecific antibody that binds to factor IX and binds to factor X (Q499-z121/J327- z119/L404- k) as described in US 2013/0330345 (comprising the amino acid sequences of sequences SEQ ID NO: 20, SEQ ID NO: 25 and SEQ ID NO: 32) and herein below abbreviated as Bsab FIX/FX, was spiked into the plasma samples in concentrations of 25, 50, 75 or 100 µg/ml resembling clinically applied concentrations of Bsab FIX/FX. The Bsab FIX/FX (Q499- z121/J327- z119/L404-k) is described in detail in US 2013/0330345 and comprises a first polypeptide comprising a first antigen-binding site that binds to blood coagulation factor IX and/or activated blood coagulation factor IX and a third polypeptide comprising a third antigen-binding site that binds to blood coagulation factor IX and/or activated blood coagulation factor IX, as well as a second polypeptide comprising a second antigen-binding site that binds to blood coagulation factor X and a fourth polypeptide comprising a fourth antigen-binding site that binds to blood coagulation factor X wherein the first polypeptide is an H chain comprising the amino acid sequence of SEQ ID NO: 20, the second polypeptide is an H chain comprising the amino acid sequence of SEQ ID NO: 25, and the third polypeptide and the fourth polypeptide are a commonly shared L chain comprising the amino acid sequence of SEQ ID NO: 32.

In addition recombinant FIX (Benefix (R)) ((non-activated) FIX) was added in vitro in part of the experiments. Alternately a commercially available prothrombin complex concentrate (PCC) comprising FIX (in non-activated form) was added in vitro in a concentration of 1 U /ml (Octaplex (R)). Octaplex is a pooled plasma coagulation factor concentrate containing coagulation factors II (220 - 760IU), VII (180 - 480IU), IX (500IU) and X (360 - 600IU). It also contains protein C, protein S, albumin, heparin and sodium citrate. As PCCs contain small amounts of heparin, which may interfere with in vitro evaluation of their activity, the PCC was incubated for 15 minutes in Heparinase solution (Hepzyme, Siemens), an enzyme, which degrades heparin in vitro.

As a control recombinant FVIII (Advate, Baxter) was added to the FVIII deficient plasma in a concentration of 100%.

The dilutions of Bsab factor IX/ factor X were prepared in 4% gelatin solution (Gelafusal).

**Table 1: tested combinations of substances.**

| FVIII dp | | |
|---|---|---|
| FVIII dp | +100% FVIII | |
| FVIII dp | +100 µg/ml Bsab FIX/FX | |
| FVIII dp | +75 µg/ml Bsab FIX/FX | |
| FVIII dp | +50 µg/ml Bsab FIX/FX | |
| FVIII dp | +25 µg/ml Bsab FIX/FX | |
| FVIII dp | +100% FVIII | +100% FIX |
| FVIII dp | +100 µg/ml Bsab FIX/FX | +100% FIX |
| FVIII dp | +75 µg/ml Bsab FIX/FX | +100% FIX |
| FVIII dp | +50 µg/ml Bsab FIX/FX | +100% FIX |
| FVIII dp | +25 µg/ml Bsab FIX/FX | +100% FIX |
| FVIII dp | +100% FVIII | +1U PCC /ml |
| FVIII dp | +100 µg/ml Bsab FIX/FX | +1U PCC /ml |
| FVIII dp | +75 µg/ml Bsab FIX/FX | +1U PCC /ml |
| FVIII dp | +25 µg/ml Bsab FIX/FX | +1U PCC /ml |

| | | |
|---|---|---|
| FVIII dp = FVIII deficient plasma | | |

Thrombin generation was continuously determined by means of a fluorogenic substrate following the activation of coagulation with a small amount of tissue factor ("PPP low reagent", instrument and all reagents by Thrombinoscope, Netherlands).

### Description of thrombin generation method:

Thrombin generation can be measured in biological plasma using the Calibrated Automated Thrombogram (CAT) method from Thrombinoscope BV, Maastricht, The Netherlands. In brief, thrombin generati6on is triggered through the extrinsic pathway of coagulation by addition of 1 pM tissue factor (TF), phospholipids and calcium ions (Ca2+). A low affinity fluorogenic substrate is added for the realtime analysis of thrombin generation. Plasma samples are calibrated against known thrombin calibrator in order to correct for the substrate depletion, sample color and inner filter effect. The fluorescence is read with a Thermo Fluoroskan. From the fluorescence signal measured the thrombin activity is calculated. The curves expressed show the free thrombin activity (y-axis, in nM thrombin) over time (x-axis, in sec).

**Control measurements: FVIII deficient plasma and FVIII deficient plasma** + **100% FVIII:**
The analysis of FVIII deficient plasma alone revealed as expected a very weak thrombin generation. This shows the physiological reason of the bleeding disorder in hemophilia A patients.

In Figure 3a, the addition of FVIII leads to a rapid thrombin generation, and in total to a 7fold thrombin generation as compared to the sample lacking FVIII.

### Addition of Bsab FIX/FX:

Also the addition of Bsab FIX/FX leads to a significant increase of the thrombin generation to a 3.4fold - 4.4 fold thrombin generation of the sample lacking FVIII.

The results are shown in Figure 3b

**Comparison of the activity of Bsab FIX/FX with the activity of FVIII on the thrombin generation of a plasma sample lacking FVIII:**
Comparing the thrombin generation of samples with the supplementation of FVIII or Bsab FIX/FX, significantly more thrombin was formed using FVIII, and also that the time to peak thrombin generation was shorter with FVIII compared to Bsab FIX/FX. ( (see Figure 3c )

**Addition of FIX to Bsab FIX/FX treated FVIII deficient plasma:** The addition of FIX (100%) to FVIII deficient plasma treated with Bsab FIX/FX leads to a significant increase of thrombin generation (see Figure 4a and Table 2).

In the sample with 75 µg Bsab FIX/FX /ml this resulted to a doubling of the thrombin generation. In the sample with 50 µg Bsab FIX/FX /ml a 75% increase of thrombin generation was determined. In the sample with 25 µg Bsab FIX/FX /ml a 50% increase of thrombin generation was found ( see Figure .... And .

In addition the time to peak was significantly shortened by the addition of FIX, i.e. thrombin generation was not only increased, but the thrombin generation was also accelerated.

As seen in Figure 4a and Table 2, both the peak thrombin generation as well as the time to peak of the samples treated with Bsab FIX/FX matched the sample with the 100% FVIII.

**Addition of prothrombin complex concentrate (PCC) to Bsab FIX/FX treated FVIII deficient plasma:**
The addition of PCC (1 U/ml) to FVIII deficient plasma treated with Bsab FIX/FX lead to a significant increase of thrombin generation.

In the sample with 75 µg Bsab FIX/FX /ml this resulted to a 94% increase of thrombin generation. In the sample with 25 µg RO5534262/ml a 90% increase of thrombin generation was found. In both cases the peak thrombin generation was similar for the Bsab FIX/FX treated samples with the addition of PCC compared to the FVIII supplemented sample. ( see Figure 4 b and Table 2)

**Table 2: Results: Induced/increased thrombin generation by tested combinations of substances in FVIII deficient plasma.**

| | | | peak height (nM) | time to peak (min) |
|---|---|---|---|---|
| FVIII dp | | | 34 | 14,0 |
| FVIII dp | +100% FVIII | | 238 | 5,7 |
| FVIII dp | +100 µg/ml Bsab FIX/FX | | 152 | 10,0 |
| FVIII dp | +75 µg/ml Bsab FIX/FX | | 124 | 11,0 |
| FVIII dp | +50 µg/ml Bsab FIX/FX | | 119 | 11,5 |
| FVIII dp | +25 µg/ml Bsab FIX/FX | | 117 | 12,0 |
| FVIII dp | +100 µg/ml Bsab FIX/FX | +100% FIX | 272 | 5,5 |
| FVIII dp | +75 µg/ml Bsab FIX/FX | +100% FIX | 247 | 6,3 |
| FVIII dp | +50 µg/ml Bsab FIX/FX | +100% FIX | 208 | 6,7 |
| FVIII dp | +25 µg/ml Bsab FIX/FX | +100% FIX | 176 | 7,5 |
| FVIII dp | +100 µg/ml Bsab FIX/FX | +1U PCC /ml | 361 | 10,7 |
| FVIII dp | +75 µg/ml Bsab FIX/FX | +1U PCC /ml | 240 | 11,0 |
| FVIII dp | +25 µg/ml Bsab FIX/FX | +1U PCC /ml | 223 | 12,0 |

Using 100 µg Bsab FIX/FX/ml and either 100% FIX or 1 U PCC/ml even a thrombin generation can be achieved which exceeds the thrombin generation found with 100% FVIII /ml. This may be desirable in case of large bleeding complications, e.g. following trauma or larger surgeries.

### Summary on the experimental data:

The experimental data shows that the addition of either FIX or PCC to plasma samples treated with Bsab FIX/FX (Q499- z121/J327- z119/L404- k) leads to a significant increase of thrombin generation in the sample.

## Claims

1. A combination of
i) a multispecific antibody that functionally substitutes for blood coagulation factor VIII which comprises a first antigen-binding site that binds to coagulation factor IX and/or activated coagulation factor IX and a second antigen-binding site that binds to coagulation factor X, and
ii) a non-activated coagulation factor IX, for use in the treatment of hemophilia A.

2. The combination for use according to claim 1,
a) for use in increasing the thrombin generation;
b) for use in increasing the thrombin generation at the site of vascular injury / at the site of tissue factor release;
c) for use in accelerating of the thrombin generation/formation;
d) for use in increasing and accelerating the thrombin generation/formation;
e) for use in accelerating the thrombin generation/formation at the site of vascular injury / at the site of tissue factor release;
f) for use in enhancing blood coagulation hemophilia A;
g) for use in enhancing fibrin clot formation; and/or
h) for preventing and/or treating bleeding, diseases accompanying bleeding, diseases caused by bleeding.

3. The combination for use according to any one of the preceding claims
a) wherein there exists an increased bleeding risk,
b) during surgery or other invasive procedures, and/or
c) after vascular injury.

4. The combination for use according to any one of the preceding claims, wherein in addition a) a coagulation factor II or b) a coagulation factor X, c) a coagulation factors II and X; or d) coagulation factors II, X and VII is/are used in the combination.

5. The combination for use according to any one of the preceding claims, wherein non-activated coagulation factor IX is comprised in a prothrombin complex concentrates (PCC).

6. The combination for use according to claim 5, wherein the prothrombin complex concentrates comprises FIX, FII, and FX.

7. The combination for use according to claim 5, wherein the prothrombin complex concentrates comprises FIX, FII, FX and FVII.

8. The combination for use according to any one of the preceding claims, wherein the antibody is bispecific and the first antigen-binding site that binds to coagulation factor IX and/or activated coagulation factor IX comprises the heavy chain CDR1, CDR2, and CDR3 amino acid sequences of SEQ ID NOs: 105, 106, and 107 (heavy chain CDRs of Q499) ), respectively, and the light chain CDR1, CDR2, and CDR3 amino acid sequences of SEQ ID NOs: 156, 157, and 158 (light chain CDR of L404), respectively.and the second antigen-binding site that binds to coagulation factor X comprises the heavy chain CDR1, CDR2, and CDR3 amino acid sequences of SEQ ID NOs: 126, 127, and 128 (heavy chain CDRs of J327), respectively, and the light chain CDR1, CDR2, and CDR3 amino acid sequences of SEQ ID NOs: 156, 157, and 158 (light chain CDR of L404), respectively.

9. The combination for use according to any one of the preceding claims, wherein the antibody is a bispecific antibody (Q499-z121/J327-z119/L404-k), comprising a) a heavy chain consisting of the amino acid sequence of SEQ ID NO: 20, b) a heavy chain consisting of the amino acid sequence of SEQ ID NO: 25, and c) a commonly shared light chain consisting of the amino acid sequence of ID NO: 32.

10. The combination for use according to any one of claims 1 to 9, wherein said multispecific antibody and FIX are co-administered simultaneously.

11. The combination for use according to any one of claims 1 to 9, wherein said multispecific antibody and FIX are co-administered sequentially.

## Patentansprüche

1. Kombination aus
i) einem multispezifischen Antikörper, der funktionell Blutgerinnungsfaktor VIII ersetzt, wobei der Antikörper eine erste Antigenbindungsstelle, die an Gerinnungsfaktor IX und/oder aktivierten Gerinnungsfaktor IX bindet, und eine zweite Antigenbindungsstelle, die an Gerinnungsfaktor X bindet, umfasst und
ii) einem nicht aktivierten Gerinnungsfaktor IX zur Verwendung bei der Behandlung von Hämophilie A.

2. Kombination zur Verwendung nach Anspruch 1,
a) zur Verwendung zum Steigern der Thrombinerzeugung;
b) zur Verwendung zum Steigern der Thrombinerzeugung an der Stelle einer Gefäßverletzung / an der Stelle einer Gewebefaktorfreisetzung;
c) zur Verwendung zum Beschleunigen der Thrombinerzeugung/-bildung;
d) zur Verwendung zum Steigern und Beschleunigen der Thrombinerzeugung/- bildung;
e) zur Verwendung zum Beschleunigen der Thrombinerzeugung/-bildung an der Stelle einer Gefäßverletzung / an der Stelle einer Gewebefaktorfreisetzung;
f) zur Verwendung zum Verstärken der Blutgerinnung bei Hämophilie A;
g) zur Verwendung zum Verstärken der Fibringerinnselbildung und/oder
h) zum Vorbeugen und/oder Behandeln von Blutung, Krankheiten in Verbindung mit Blutung, Krankheiten, die durch Blutung verursacht werden.

3. Kombination zur Verwendung nach einem der vorstehenden Ansprüche,
a) wobei ein erhöhtes Blutungsrisiko besteht,
b) während eines chirurgischen Eingriffs oder anderer invasiver Prozeduren und/oder
c) nach einer Gefäßverletzung.

4. Kombination zur Verwendung nach einem der vorstehenden Ansprüche, wobei zusätzlich a) ein Gerinnungsfaktor II oder b) ein Gerinnungsfaktor X, c) ein Gerinnungsfaktoren II und X oder d) Gerinnungsfaktoren II, X und VII in der Kombination verwendet wird/werden.

5. Kombination zur Verwendung nach einem der vorstehenden Ansprüche, wobei nicht aktivierter Gerinnungsfaktor IX in Prothrombin-Komplex-Konzentraten (PCC) enthalten ist.

6. Kombination zur Verwendung nach Anspruch 5, wobei die Prothrombin-Komplex-Konzentrate FIX, FII, und FX umfasst.

7. Kombination zur Verwendung nach Anspruch 5, wobei die Prothrombin-Komplex-Konzentrate FIX, FII, FX und FVII umfasst.

8. Kombination zur Verwendung nach einem der vorstehenden Ansprüche, wobei der Antikörper bispezifisch ist und die erste Antigenbindungsstelle, die an Gerinnungsfaktor IX und/oder aktivierten Gerinnungsfaktor IX bindet, die Aminosäuresequenzen von SEQ ID NO:105, 106 und 107 von CDR1, CDR2 bzw. CDR3 der schweren Kette (CDRs der schwere Kette von Q499) ) und die Aminosäuresequenzen von SEQ ID NO:156, 157 und 158 von CDR1, CDR2 bzw. CDR3 der leichten Kette (CDR der leichten Kette von L404) umfasst, und die zweite Antigenbindungsstelle, die an Gerinnungsfaktor X bindet, die Aminosäuresequenzen von SEQ ID NO:126, 127 und 128 von CDR1, CDR2 bzw. CDR3 der schweren Kette (CDRs der schweren Kette von J327) und die Aminosäuresequenzen von SEQ ID NO:156, 157 und 158 von CDR1, CDR2 bzw. CDR3 der leichten Kette (CDR der leichten Kette von L404) umfasst.

9. Kombination zur Verwendung nach einem der vorstehenden Ansprüche, wobei der Antikörper ein bispezifischer Antikörper ist (Q499-z121/J327-z119/L404-k), der a) eine schwere Kette, bestehend aus der Aminosäuresequenz von SEQ ID NO:20, b) eine schwere Kette, bestehend aus der Aminosäuresequenz von SEQ ID NO:25, und c) eine gemeinsame geteilte leichte Kette, bestehend aus der Aminosäuresequenz von ID NO:32, umfasst.

10. Kombination zur Verwendung nach einem der Ansprüche 1 bis 9, wobei der multispezifische Antikörper und FIX gleichzeitig gemeinsam verabreicht werden.

11. Kombination zur Verwendung nach einem der Ansprüche 1 bis 9, wobei der multispezifische Antikörper und FIX sequentiell gemeinsam verabreicht werden.

## Revendications

1. Combinaison de
i) un anticorps multispécifique qui se substitue de manière fonctionnelle au facteur de coagulation sanguine VIII qui comprend un premier site de liaison à l'antigène qui se lie au facteur de coagulation IX et/ou au facteur de coagulation IX activé et un second site de liaison à l'antigène qui se lie au facteur de coagulation X, et
ii) un facteur de coagulation IX non activé, pour une utilisation dans le traitement de l'hémophilie A.

2. Combinaison pour une utilisation selon la revendication 1,
a) pour une utilisation dans l'augmentation de la génération de thrombine ;
b) pour une utilisation dans l'augmentation de la génération de thrombine au niveau du site de lésion vasculaire/au niveau du site de libération de facteur tissulaire ;
c) pour une utilisation dans l'accélération de la génération/formation de thrombine ;
d) pour une utilisation dans l'augmentation et l'accélération de la génération/formation de thrombine ;
e) pour une utilisation dans l'accélération de la génération/formation de thrombine au niveau du site de lésion vasculaire/au niveau du site de libération de facteur tissulaire ;
f) pour une utilisation dans l'amélioration de la coagulation sanguine l'hémophilie A;
g) pour une utilisation dans l'amélioration de la formation de caillots de fibrine ; et/ou
h) pour la prévention et/ou le traitement des saignements, des maladies accompagnant les saignements, des maladies provoquées par les saignements.

3. Combinaison pour une utilisation selon l'une quelconque des revendications précédentes
a) dans laquelle il existe un risque accru de saignement,
b) pendant une opération chirurgicale ou d'autres procédures invasives, et/ou
c) après une lésion vasculaire.

4. Combinaison pour une utilisation selon l'une quelconque des revendications précédentes, dans laquelle en outre a) un facteur de coagulation II ou b) un facteur de coagulation X, c) un facteurs de coagulation II et X ; ou d) des facteurs de coagulation II, X et VII est/sont utilisé(s) dans la combinaison.

5. Combinaison pour une utilisation selon l'une quelconque des revendications précédentes, dans laquelle le facteur de coagulation IX non activé est compris dans des concentrés de complexe prothrombinique (CCP).

6. Combinaison pour une utilisation selon la revendication 5, dans laquelle les concentrés de complexe prothrombinique comprend FIX, FII et FX.

7. Combinaison pour une utilisation selon la revendication 5, dans laquelle les concentrés de complexe prothrombinique comprend FIX, FII, FX et FVII.

8. Combinaison pour une utilisation selon l'une quelconque des revendications précédentes, dans laquelle l'anticorps est bispécifique et le premier site de liaison à l'antigène qui se lie au facteur de coagulation IX et/ou au facteur de coagulation IX activé comprend les séquences d'acides aminés de CDR1, CDR2 et CDR3 de chaîne lourde de SEQ ID NO : 105, 106 et 107 (CDR de chaîne lourde de Q499)), respectivement, et les séquences d'acides aminés de CDR1, CDR2 et CDR3 de chaîne légère de SEQ ID NO : 156, 157 et 158 (CDR de chaîne légère de L404), respectivement, et le second site de liaison à l'antigène qui se lie au facteur de coagulation X comprend les séquences d'acides aminés de CDR1, CDR2 et CDR3 de chaîne lourde de SEQ ID NO : 126, 127 et 128 (CDR de chaîne lourde de J327), respectivement, et les séquences d'acides aminés de CDR1, CDR2 et CDR3 de chaîne légère de SEQ ID NO : 156, 157 et 158 (CDR de chaîne légère de L404), respectivement.

9. Combinaison pour une utilisation selon l'une quelconque des revendications précédentes, dans laquelle l'anticorps est un anticorps bispécifique (Q499-z121/J327-z119/L404-k), comprenant a) une chaîne lourde constituée de la séquence d'acides aminés de SEQ ID NO : 20, b) une chaîne lourde constituée de la séquence d'acides aminés de SEQ ID NO : 25, et c) une chaîne légère communément partagée constituée de la séquence d'acides aminés de ID NO : 32.

10. Combinaison pour une utilisation selon l'une quelconque des revendications 1 à 9, dans laquelle ledit anticorps multispécifique et FIX sont co-administrés simultanément.

11. Combinaison pour une utilisation selon l'une quelconque des revendications 1 à 9, dans laquelle ledit anticorps multispécifique et FIX sont co-administrés séquentiellement.


### SEQUENCE LISTING

<110> F.Hoffmann La-Roche AG
<120> Combination therapy with coagulation factors and multispecific antibodies
<130> P32729-WO
<150> EP15164045.5
   <151> 2015-04-17
<160> 179
<170> PatentIn version 3.5
<210> 1
   <211> 448
   <212> PRT
   <213> Artificial
<220>
   <223> artificial sequence
<400> 1
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Met Ala Trp Val Arg Gln Ala Pro Gly Lys Gly Leu Glu Trp Val
           35                  40                  45
   Ala Ser Ile Ser Pro Ser Gly Gly Ser Thr Tyr Tyr Arg His Ser Val
       50                  55                  60
   Lys Gly Arg Phe Thr Val Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ala Gly His Asn Leu Gly Ala Gly Trp Tyr Phe Asp Phe
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Cys
               340                 345                 350
   Thr Leu Pro Pro Ser Gln Glu Glu Met Thr Lys Asn Gln Val Ser Leu
           355                 360                 365
   Trp Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Arg Leu Thr Val Asp
                   405                 410                 415
   Lys Ser Arg Trp Gln Glu Gly Asn Val Phe Ser Cys Ser Val Met His
               420                 425                 430
   Glu Ala Leu His Asn His Tyr Thr Gln Lys Ser Leu Ser Leu Ser Leu
           435                 440                 445
<210> 2
   <211> 448
   <212> PRT
   <213> Artificial
<220>
   <223> artificial sequence
<400> 2
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Met Ala Trp Val Arg Gln Ala Pro Gly Lys Gly Leu Glu Trp Val
           35                  40                  45
   Ala Ser Ile Ser Pro Ser Gly Gly Ser Thr Tyr Tyr Arg Arg Ser Val
       50                  55                  60
   Lys Gly Arg Phe Thr Val Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ala Gly His Asn Leu Gly Ala Gly Trp Tyr Phe Asp Phe
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Lys Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Arg Leu Thr Val Asp
                   405                 410                 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 3
   <211> 448
   <212> PRT
   <213> Artificial
<220>
   <223> artificial sequence
<400> 3
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Met Ala Trp Val Arg Gln Ala Pro Gly Lys Gly Leu Glu Trp Val
           35                  40                  45
   Ala Ser Ile Ser Pro Ser Gly Gly Ser Thr Tyr Tyr Arg Arg Ser Val
       50                  55                  60
   Lys Gly Arg Phe Thr Val Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ala Gly His Asn Phe Gly Ala Gly Trp Tyr Phe Asp Phe
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
   180 185 190
   Thr Val Pro Ser Ser Ser Leu Gly Thr Lys Thr Tyr Thr Cys Asn Val
   195 200 205
   Asp His Lys Pro Ser Asn Thr Lys Val Asp Lys Arg Val Glu Ser Lys
   210 215 220
   Tyr Gly Pro Pro Cys Pro Ser Cys Pro Ala Pro Glu Phe Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser Gln Glu
   260 265 270
   Asp Pro Glu Val Gln Phe Asn Trp Tyr Val Asp Gly Val Glu Val His
   275 280 285
   Asn Ala Lys Thr Lys Pro Arg Glu Glu Gln Phe Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Tyr
   340 345 350
   Thr Leu Pro Pro Ser Gln Lys Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Arg Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 4
   <211> 444
   <212> PRT
   <213> Artificial
<220>
   <223> artificial sequence
<400> 4
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Gly Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Arg Gly Tyr His Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Lys Thr Tyr Thr Cys Asn Val Asp His Lys Pro
   195 200 205
   Ser Asn Thr Lys Val Asp Lys Arg Val Glu Ser Lys Tyr Gly Pro Pro
   210 215 220
   Cys Pro Pro Cys Pro Ala Pro Glu Phe Leu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser Gln Glu Asp Pro Glu Val
   260 265 270
   Gln Phe Asn Trp Tyr Val Asp Gly Val Glu Val His Asn Ala Lys Thr
   275 280 285
   Lys Pro Arg Glu Glu Gln Phe Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Gly Leu Pro Ser Ser Ile Glu Lys Thr Ile Ser
   325 330 335
   Lys Ala Lys Gly Gln Pro Arg Glu Pro Gln Val Tyr Thr Leu Pro Pro
   340 345 350
   Ser Gln Cys Glu Met Thr Lys Asn Gln Val Ser Leu Ser Cys Ala Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Val Ser Arg Leu Thr Val Asp Lys Ser Arg Trp
   405 410 415
   Gln Glu Gly Asn Val Phe Ser Cys Ser Val Met His Glu Ala Leu His
   420 425 430
   Asn His Tyr Thr Gln Lys Ser Leu Ser Leu Ser Leu
   435 440
<210> 5
   <211> 444
   <212> PRT
   <213> Artificial
<220>
   <223> artificial sequence
<400> 5
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met His Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Tyr His Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Lys Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Phe Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Cys Glu Met Thr Lys Asn Gln Val Ser Leu Ser Cys Ala Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Val Ser Arg Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn His Tyr Thr Gln Lys Ser Leu Ser Leu Ser Leu
           435                 440
<210> 6
   <211> 444
   <212> PRT
   <213> Artificial
<220>
   <223> artificial sequence
<400> 6
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Asn His Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn Arg Tyr Thr Gln Glu Ser Leu Ser Leu Ser Pro
           435                 440
<210> 7
   <211> 444
   <212> PRT
   <213> Artificial
<220>
   <223> artificial sequence
<400> 7
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Lys Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Thr Tyr His Cys
                   85                  90                  95
   Ala Arg Arg Gln Ser Tyr Gly Tyr His Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn Arg Tyr Thr Gln Glu Ser Leu Ser Leu Ser Pro
           435                 440
<210> 8
   <211> 213
   <212> PRT
   <213> Artificial
<220>
   <223> artificial sequence
<400> 8
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Ile Tyr Lys Asn
               20                  25                  30
   Leu Ala Trp Tyr Gln Gln Lys Pro Gly Gln Ala Pro Lys Leu Leu Ile
           35                  40                  45
   Tyr Ser Ala Ser Tyr Arg Tyr Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Tyr Ser Gly Leu Thr
                   85                  90                  95
   Phe Gly Gly Gly Thr Lys Val Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 9
   <211> 214
   <212> PRT
   <213> Artificial
<220>
   <223> artificial sequence
<400> 9
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Ile Tyr Lys Asn
               20                  25                  30
   Leu Ala Trp Tyr Gln Gln Lys Pro Gly Gln Ala Pro Lys Leu Leu Ile
           35                  40                  45
   Tyr Ser Ala Ser Tyr Arg Tyr Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Tyr Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 10
   <211> 448
   <212> PRT
   <213> Artificial
<220>
   <223> artificial sequence
<400> 10
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Met Ala Trp Val Arg Gln Ala Pro Gly Lys Gly Leu Glu Trp Val
           35                  40                  45
   Ala Ser Ile Ser Pro Ser Gly Gly Ser Thr Tyr Tyr Arg Arg Ser Val
       50                  55                  60
   Lys Gly Arg Phe Thr Val Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ala Gly His Asn Phe Gly Ala Gly Trp Tyr Phe Asp Phe
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Lys Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Arg Leu Thr Val Asp
                   405                 410                 415
   Lys Ser Arg Trp Gln Glu Gly Asn Val Phe Ser Cys Ser Val Met His
               420                 425                 430
   Glu Ala Leu His Asn His Tyr Thr Gln Lys Ser Leu Ser Leu Ser Leu
           435                 440                 445
<210> 11
   <211> 448
   <212> PRT
   <213> Artificial
<220>
   <223> artificial sequence
<400> 11
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Met Ala Trp Val Arg Gln Ala Pro Gly Lys Gly Leu Glu Trp Val
           35                  40                  45
   Ala Ser Ile Ser Pro Ser Gly Gly Ser Thr Tyr Tyr Arg Arg Ser Val
       50                  55                  60
   Lys Gly Arg Phe Thr Val Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ala Gly His Asn Tyr Gly Ala Gly Trp Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Cys
               340                 345                 350
   Thr Leu Pro Pro Ser Gln Glu Glu Met Thr Lys Asn Gln Val Ser Leu
           355                 360                 365
   Trp Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Arg Leu Thr Val Asp
                   405                 410                 415
   Lys Ser Arg Trp Gln Glu Gly Asn Val Phe Ser Cys Ser Val Met His
               420                 425                 430
   Glu Ala Leu His Asn His Tyr Thr Gln Lys Ser Leu Ser Leu Ser Leu
           435                 440                 445
<210> 12
   <211> 448
   <212> PRT
   <213> Artificial
<220>
   <223> artificial sequence
<400> 12
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Ile Asn Trp Val Arg Gln Ala Pro Gly Lys Gly Leu Glu Trp Val
           35                  40                  45
   Ala Ser Ile Ser Pro Ser Gly Gly Ser Thr Tyr Tyr Arg Arg Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Thr Arg Ala Gly His Asn Tyr Gly Ala Gly Trp Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
   275 280 285
   Asn Ala Lys Thr Lys Pro Arg Glu Glu Gln Phe Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Cys
   340 345 350
   Thr Leu Pro Pro Ser Gln Glu Glu Met Thr Lys Asn Gln Val Ser Leu
   355 360 365
   Trp Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Arg Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 13
   <211> 448
   <212> PRT
   <213> Artificial
<220>
   <223> artificial sequence
<400> 13
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Asn Trp Val Arg Gln Ala Pro Gly Lys Gly Leu Glu Trp Val
           35                  40                  45
   Ser Ser Ile Ser Pro Ser Gly Gln Ser Thr Tyr Tyr Arg Arg Glu Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ser Gly His Asn Tyr Gly Gly Gly Trp Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Tyr
   340 345 350
   Thr Leu Pro Pro Ser Gln Lys Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 14
   <211> 448
   <212> PRT
   <213> Artificial
<220>
   <223> artificial sequence
<400> 14
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ser Gly His Asn Tyr Gly Gly Gly Trp Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Cys
   340 345 350
   Thr Leu Pro Pro Ser Gln Glu Glu Met Thr Lys Asn Gln Val Ser Leu
   355 360 365
   Trp Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Arg Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 15
   <211> 448
   <212> PRT
   <213> Artificial
<220>
   <223> artificial sequence
<400> 15
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ser Gly His Asn Tyr Gly Gly Gly Trp Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Lys Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Glu Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
<210> 16
   <211> 448
   <212> PRT
   <213> Artificial
<220>
   <223> artificial sequence
<400> 16
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Ile Ser Trp Val Arg Gln Ala Pro Gly Lys Gly Leu Glu Trp Val
           35                  40                  45
   Ser Ser Ile Ser Pro Ser Gly Gln Ser Thr Tyr Tyr Arg Arg Glu Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ser Gly His Asn Phe Gly Gly Gly Trp Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Cys
   340 345 350
   Thr Leu Pro Pro Ser Gln Glu Glu Met Thr Lys Asn Gln Val Ser Leu
   355 360 365
   Trp Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Arg Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 17
   <211> 448
   <212> PRT
   <213> Artificial
<220>
   <223> artificial sequence
<400> 17
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly Lys Ser Tyr Gly Gly Gly Trp Tyr Phe Asp Tyr
   100 105 110
   Trp Gly Gln Gly Thr Leu Val Thr Val Ser Ser Ala Ser Thr Lys Gly
   115 120 125
   Pro Ser Val Phe Pro Leu Ala Pro Cys Ser Arg Ser Thr Ser Glu Ser
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Thr Cys Asn Val
   195 200 205
   Asp His Lys Pro Ser Asn Thr Lys Val Asp Lys Arg Val Glu Ser Lys
   210 215 220
   Tyr Gly Pro Pro Cys Pro Pro Cys Pro Ala Pro Glu Phe Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser Gln Glu
   260 265 270
   Asp Pro Glu Val Gln Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Tyr
   340 345 350
   Thr Leu Pro Pro Ser Gln Lys Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
<210> 18
   <211> 448
   <212> PRT
   <213> Artificial
<220>
   <223> artificial sequence
<400> 18
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly Arg Glu Tyr Gly Gly Gly Trp Tyr Phe Asp Tyr
   100 105 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Lys Glu Met Thr Lys Asn Gln Val Ser Leu
           355                 360                 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Glu Gly Asn Val Phe Ser Cys Ser Val Met His
   420 425 430
   Glu Ala Leu His Asn Arg Tyr Thr Gln Lys Ser Leu Ser Leu Ser Pro
   435 440 445
<210> 19
   <211> 448
   <212> PRT
   <213> Artificial
<220>
   <223> artificial sequence
<400> 19
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Thr Gly Arg Glu Tyr Gly Gly Gly Trp Tyr Phe Asp Tyr
   100 105 110
   Trp Gly Gln Gly Thr Leu Val Thr Val Ser Ser Ala Ser Thr Lys Gly
   115 120 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Lys Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Pro
   435 440 445
<210> 20
   <211> 448
   <212> PRT
   <213> Artificial
<220>
   <223> artificial sequence
<400> 20
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Thr Gly Arg Glu Tyr Gly Gly Gly Trp Tyr Phe Asp Tyr
   100 105 110
   Trp Gly Gln Gly Thr Leu Val Thr Val Ser Ser Ala Ser Thr Lys Gly
   115 120 125
   Pro Ser Val Phe Pro Leu Ala Pro Cys Ser Arg Ser Thr Ser Glu Ser
   130 135 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Lys Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
   405 410 415
   Lys Ser Arg Trp Gln Glu Gly Asn Val Phe Ser Cys Ser Val Met His
   420 425 430
   Glu Ala Leu His Asn Arg Tyr Thr Gln Lys Ser Leu Ser Leu Ser Pro
   435 440 445
<210> 21
   <211> 444
   <212> PRT
   <213> Artificial
<220>
   <223> artificial sequence
<400> 21
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Lys Ser Thr Gly Thr Ala Tyr
   65 70 75 80
   Met Glu Leu Ser Ser Leu Arg Ser Glu Asp Thr Ala Val Tyr Tyr Cys
   85 90 95
   Ala Arg Arg Lys Ser Tyr Gly Tyr His Leu Asp Glu Trp Gly Glu Gly
   100 105 110
   Thr Leu Val Thr Val Ser Ser Ala Ser Thr Lys Gly Pro Ser Val Phe
   115 120 125
   Pro Leu Ala Pro Cys Ser Arg Ser Thr Ser Glu Ser Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Lys Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Phe Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Cys Glu Met Thr Lys Asn Gln Val Ser Leu Ser Cys Ala Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Val Ser Arg Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn His Tyr Thr Gln Lys Ser Leu Ser Leu Ser Leu
           435                 440
<210> 22
   <211> 444
   <212> PRT
   <213> Artificial
<220>
   <223> artificial sequence
<400> 22
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Gly Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Tyr Tyr Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn Arg Tyr Thr Gln Glu Ser Leu Ser Leu Ser Pro
   435 440
<210> 23
   <211> 444
   <212> PRT
   <213> Artificial
<220>
   <223> artificial sequence
<400> 23
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Lys Ser Thr Asp Thr Ala Tyr
   65 70 75 80
   Met Glu Leu Ser Ser Leu Arg Ser Glu Asp Thr Ala Val Tyr His Cys
   85 90 95
   Ala Arg Arg Lys Ser Tyr Gly Tyr Tyr Leu Asp Glu Trp Gly Glu Gly
   100 105 110
   Thr Leu Val Thr Val Ser Ser Ala Ser Thr Lys Gly Pro Ser Val Phe
   115 120 125
   Pro Leu Ala Pro Cys Ser Arg Ser Thr Ser Glu Ser Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn Arg Tyr Thr Gln Glu Ser Leu Ser Leu Ser Pro
           435                 440
<210> 24
   <211> 444
   <212> PRT
   <213> Artificial
<220>
   <223> artificial sequence
<400> 24
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Thr Tyr His Cys
                   85                  90                  95
   Ala Arg Arg Lys Ser Tyr Gly Tyr Tyr Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn Arg Tyr Thr Gln Glu Ser Leu Ser Leu Ser Pro
           435                 440
<210> 25
   <211> 444
   <212> PRT
   <213> Artificial
<220>
   <223> artificial sequence
<400> 25
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Thr Tyr His Cys
                   85                  90                  95
   Ala Arg Arg Lys Ser Tyr Gly Tyr Tyr Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn His Tyr Thr Gln Glu Ser Leu Ser Leu Ser Pro
           435                 440
<210> 26
   <211> 444
   <212> PRT
   <213> Artificial
<220>
   <223> artificial sequence
<400> 26
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Thr Ser Thr Asp Thr Ala Tyr
   65                  70                  75                  80
   Met Glu Leu Ser Ser Leu Arg Ser Glu Asp Thr Ala Thr Tyr His Cys
                   85                  90                  95
   Ala Arg Arg Lys Ser Tyr Gly Tyr His Leu Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn His Tyr Thr Gln Glu Ser Leu Ser Leu Ser Pro
           435                 440
<210> 27
   <211> 444
   <212> PRT
   <213> Artificial
<220>
   <223> artificial sequence
<400> 27
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Lys Ser Thr Asp Thr Ala Tyr
   65 70 75 80
   Met Glu Leu Ser Ser Leu Arg Ser Glu Asp Thr Ala Thr Tyr His Cys
   85 90 95
   Ala Arg Arg Lys Ser Tyr Gly Tyr His Leu Asp Glu Trp Gly Glu Gly
   100 105 110
   Thr Leu Val Thr Val Ser Ser Ala Ser Thr Lys Gly Pro Ser Val Phe
   115 120 125
   Pro Leu Ala Pro Cys Ser Arg Ser Thr Ser Glu Ser Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Thr Cys Asn Val Asp His Lys Pro
   195 200 205
   Ser Asn Thr Lys Val Asp Lys Arg Val Glu Ser Lys Tyr Gly Pro Pro
   210 215 220
   Cys Pro Pro Cys Pro Ala Pro Glu Phe Leu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser Gln Glu Asp Pro Glu Val
   260 265 270
   Gln Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Gly Leu Pro Ser Ser Ile Glu Lys Thr Ile Ser
   325 330 335
   Lys Ala Lys Gly Gln Pro Arg Glu Pro Gln Val Tyr Thr Leu Pro Pro
   340 345 350
   Ser Gln Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Glu Gly Asn Val Phe Ser Cys Ser Val Met His Glu Ala Leu His
   420 425 430
   Asn Arg Tyr Thr Gln Glu Ser Leu Ser Leu Ser Pro
   435 440
<210> 28
   <211> 214
   <212> PRT
   <213> Artificial
<220>
   <223> artificial sequence
<400> 28
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Lys Asn Ile Glu Arg Asn
               20                  25                  30
   Leu Ala Trp Tyr Gln Gln Lys Pro Gly Gln Ala Pro Arg Leu Leu Ile
           35                  40                  45
   Tyr Arg Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 29
   <211> 214
   <212> PRT
   <213> Artificial
<220>
   <223> artificial sequence
<400> 29
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Arg Ala Asp Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 30
   <211> 214
   <212> PRT
   <213> Artificial
<220>
   <223> artificial sequence
<400> 30
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 31
   <211> 214
   <212> PRT
   <213> Artificial
<220>
   <223> artificial sequence
<400> 31
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 32
   <211> 214
   <212> PRT
   <213> Artificial
<220>
   <223> artificial sequence
<400> 32
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Asp Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 33
   <211> 214
   <212> PRT
   <213> Artificial
<220>
   <223> artificial sequence
<400> 33
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Arg Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Asp Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
                               210
<210> 34
   <211> 214
   <212> PRT
   <213> Artificial
<220>
   <223> artificial sequence
<400> 34
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Arg Ala Asp Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Asp Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
   210
<210> 35
   <211> 123
   <212> PRT
   <213> Artificial
<220>
   <223> artificial sequence
<400> 35
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Met Ala Trp Val Arg Gln Ala Pro Gly Lys Gly Leu Glu Trp Val
   35 40 45
   Ala Ser Ile Ser Pro Ser Gly Gly Ser Thr Tyr Tyr Arg His Ser Val
   50 55 60
   Lys Gly Arg Phe Thr Val Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Arg Ala Gly His Asn Leu Gly Ala Gly Trp Tyr Phe Asp Phe
   100 105 110
   Trp Gly Gln Gly Thr Leu Val Thr Val Ser Ser
   115 120
<210> 36
   <211> 123
   <212> PRT
   <213> Artificial
<220>
   <223> artificial sequence
<400> 36
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Met Ala Trp Val Arg Gln Ala Pro Gly Lys Gly Leu Glu Trp Val
   35 40 45
   Ala Ser Ile Ser Pro Ser Gly Gly Ser Thr Tyr Tyr Arg Arg Ser Val
   50 55 60
   Lys Gly Arg Phe Thr Val Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Arg Ala Gly His Asn Leu Gly Ala Gly Trp Tyr Phe Asp Phe
   100 105 110
   Trp Gly Gln Gly Thr Leu Val Thr Val Ser Ser
   115 120
<210> 37
   <211> 123
   <212> PRT
   <213> Artificial
<220>
   <223> artificial sequence
<400> 37
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Met Ala Trp Val Arg Gln Ala Pro Gly Lys Gly Leu Glu Trp Val
   35 40 45
   Ala Ser Ile Ser Pro Ser Gly Gly Ser Thr Tyr Tyr Arg Arg Ser Val
   50 55 60
   Lys Gly Arg Phe Thr Val Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Arg Ala Gly His Asn Phe Gly Ala Gly Trp Tyr Phe Asp Phe
   100 105 110
   Trp Gly Gln Gly Thr Leu Val Thr Val Ser Ser
   115 120
<210> 38
   <211> 123
   <212> PRT
   <213> Artificial
<220>
   <223> artificial sequence
<400> 38
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Met Ala Trp Val Arg Gln Ala Pro Gly Lys Gly Leu Glu Trp Val
   35 40 45
   Ala Ser Ile Ser Pro Ser Gly Gly Ser Thr Tyr Tyr Arg Arg Ser Val
   50 55 60
   Lys Gly Arg Phe Thr Val Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Arg Ala Gly His Asn Tyr Gly Ala Gly Trp Tyr Phe Asp Tyr
   100 105 110
   Trp Gly Gln Gly Thr Leu Val Thr Val Ser Ser
   115 120
<210> 39
   <211> 123
   <212> PRT
   <213> Artificial
<220>
   <223> artificial sequence
<400> 39
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Asn Trp Val Arg Gln Ala Pro Gly Lys Gly Leu Glu Trp Val
   35 40 45
   Ala Ser Ile Ser Pro Ser Gly Gly Ser Thr Tyr Tyr Arg Arg Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Thr Arg Ala Gly His Asn Tyr Gly Ala Gly Trp Tyr Phe Asp Tyr
   100 105 110
   Trp Gly Gln Gly Thr Leu Val Thr Val Ser Ser
   115 120
<210> 40
   <211> 123
   <212> PRT
   <213> Artificial
<220>
   <223> artificial sequence
<400> 40
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Asn Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly His Asn Tyr Gly Gly Gly Trp Tyr Phe Asp Tyr
   100 105 110
   Trp Gly Gln Gly Thr Leu Val Thr Val Ser Ser
   115 120
<210> 41
   <211> 123
   <212> PRT
   <213> Artificial
<220>
   <223> artificial sequence
<400> 41
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly His Asn Tyr Gly Gly Gly Trp Tyr Phe Asp Tyr
   100 105 110
   Trp Gly Gln Gly Thr Leu Val Thr Val Ser Ser
   115 120
<210> 42
   <211> 123
   <212> PRT
   <213> Artificial
<220>
   <223> artificial sequence
<400> 42
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Ser Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly His Asn Phe Gly Gly Gly Trp Tyr Phe Asp Tyr
   100 105 110
   Trp Gly Gln Gly Thr Leu Val Thr Val Ser Ser
   115 120
<210> 43
   <211> 123
   <212> PRT
   <213> Artificial
<220>
   <223> artificial sequence
<400> 43
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly Lys Ser Tyr Gly Gly Gly Trp Tyr Phe Asp Tyr
   100 105 110
   Trp Gly Gln Gly Thr Leu Val Thr Val Ser Ser
   115 120
<210> 44
   <211> 123
   <212> PRT
   <213> Artificial
<220>
   <223> artificial sequence
<400> 44
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ser Gly Arg Glu Tyr Gly Gly Gly Trp Tyr Phe Asp Tyr
   100 105 110
   Trp Gly Gln Gly Thr Leu Val Thr Val Ser Ser
   115 120
<210> 45
   <211> 123
   <212> PRT
   <213> Artificial
<220>
   <223> artificial sequence
<400> 45
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Thr Gly Arg Glu Tyr Gly Gly Gly Trp Tyr Phe Asp Tyr
   100 105 110
   Trp Gly Gln Gly Thr Leu Val Thr Val Ser Ser
   115 120
<210> 46
   <211> 119
   <212> PRT
   <213> Artificial
<220>
   <223> artificial sequence
<400> 46
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Gly Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Lys Ser Tyr Gly Tyr His Leu Asp Glu Trp Gly Glu Gly
               100                 105                 110
   Thr Leu Val Thr Val Ser Ser
           115
<210> 47
   <211> 119
   <212> PRT
   <213> Artificial
<220>
   <223> artificial sequence
<400> 47
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Lys Ser Thr Gly Thr Ala Tyr
   65 70 75 80
   Met Glu Leu Ser Ser Leu Arg Ser Glu Asp Thr Ala Val Tyr His Cys
   85 90 95
   Ala Arg Arg Lys Ser Tyr Gly Tyr Tyr Leu Asp Glu Trp Gly Glu Gly
   100 105 110
   Thr Leu Val Thr Val Ser Ser
   115
<210> 48
   <211> 119
   <212> PRT
   <213> Artificial
<220>
   <223> artificial sequence
<400> 48
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
                       20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Gly Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Arg Gly Tyr His Leu Asp Glu Trp Gly Glu Gly
               100                 105                 110
   Thr Leu Val Thr Val Ser Ser
           115
<210> 49
   <211> 119
   <212> PRT
   <213> Artificial
<220>
   <223> artificial sequence
<400> 49
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Tyr Tyr Leu Asp Glu Trp Gly Glu Gly
               100                 105                 110
   Thr Leu Val Thr Val Ser Ser
           115
<210> 50
   <211> 119
   <212> PRT
   <213> Artificial
<220>
   <223> artificial sequence
<400> 50
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met His Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Tyr His Leu Asp Glu Trp Gly Glu Gly
               100                 105                 110
   Thr Leu Val Thr Val Ser Ser
           115
<210> 51
   <211> 119
   <212> PRT
   <213> Artificial
<220>
   <223> artificial sequence
<400> 51
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
                   20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Asn His Leu Asp Glu Trp Gly Glu Gly
               100                 105                 110
   Thr Leu Val Thr Val Ser Ser
           115
<210> 52
   <211> 119
   <212> PRT
   <213> Artificial
<220>
   <223> artificial sequence
<400> 52
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Thr Tyr His Cys
                   85                  90                  95
   Ala Arg Arg Lys Ser Tyr Gly Tyr Tyr Leu Asp Glu Trp Gly Glu Gly
               100                 105                 110
   Thr Leu Val Thr Val Ser Ser
           115
<210> 53
   <211> 119
   <212> PRT
   <213> Artificial
<220>
   <223> artificial sequence
<400> 53
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Thr Ser Thr Asp Thr Ala Tyr
   65 70 75 80
   Met Glu Leu Ser Ser Leu Arg Ser Glu Asp Thr Ala Thr Tyr His Cys
   85 90 95
   Ala Arg Arg Lys Ser Tyr Gly Tyr His Leu Asp Glu Trp Gly Glu Gly
   100 105 110
   Thr Leu Val Thr Val Ser Ser
   115
<210> 54
   <211> 119
   <212> PRT
   <213> Artificial
<220>
   <223> artificial sequence
<400> 54
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Lys Ser Gly Gly Ser Ile Tyr Asn Glu Glu Phe
   50 55 60
   Gln Asp Arg Val Ile Met Thr Val Asp Lys Ser Thr Asp Thr Ala Tyr
   65 70 75 80
   Met Glu Leu Ser Ser Leu Arg Ser Glu Asp Thr Ala Thr Tyr His Cys
   85 90 95
   Ala Arg Arg Gln Ser Tyr Gly Tyr His Leu Asp Glu Trp Gly Glu Gly
   100 105 110
   Thr Leu Val Thr Val Ser Ser
   115
<210> 55
   <211> 119
   <212> PRT
   <213> Artificial
<220>
   <223> artificial sequence
<400> 55
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Lys Ser Thr Asp Thr Ala Tyr
   65 70 75 80
   Met Glu Leu Ser Ser Leu Arg Ser Glu Asp Thr Ala Thr Tyr His Cys
   85 90 95
   Ala Arg Arg Lys Ser Tyr Gly Tyr His Leu Asp Glu Trp Gly Glu Gly
   100 105 110
   Thr Leu Val Thr Val Ser Ser
   115
<210> 56
   <211> 106
   <212> PRT
   <213> Artificial
<220>
   <223> artificial sequence
<400> 56
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Ile Tyr Lys Asn
               20                  25                  30
   Leu Ala Trp Tyr Gln Gln Lys Pro Gly Gln Ala Pro Lys Leu Leu Ile
           35                  40                  45
   Tyr Ser Ala Ser Tyr Arg Tyr Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Tyr Ser Gly Leu Thr
                   85                  90                  95
   Phe Gly Gly Gly Thr Lys Val Glu Ile Lys
               100                 105
<210> 57
   <211> 107
   <212> PRT
   <213> Artificial
<220>
   <223> artificial sequence
<400> 57
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Ile Tyr Lys Asn
               20                  25                  30
   Leu Ala Trp Tyr Gln Gln Lys Pro Gly Gln Ala Pro Lys Leu Leu Ile
           35                  40                  45
   Tyr Ser Ala Ser Tyr Arg Tyr Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Tyr Ser Pro Pro Leu
                   85                  90                  95
   Thr Phe Gly Gly Gly Thr Lys Val Glu Ile Lys
               100                 105
<210> 58
   <211> 107
   <212> PRT
   <213> Artificial
<220>
   <223> artificial sequence
<400> 58
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Lys Asn Ile Glu Arg Asn
               20                  25                  30
   Leu Ala Trp Tyr Gln Gln Lys Pro Gly Gln Ala Pro Arg Leu Leu Ile
           35                  40                  45
   Tyr Arg Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys
               100                 105
<210> 59
   <211> 107
   <212> PRT
   <213> Artificial
<220>
   <223> artificial sequence
<400> 59
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Arg Ala Asp Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys
               100                 105
<210> 60
   <211> 107
   <212> PRT
   <213> Artificial
<220>
   <223> artificial sequence
<400> 60
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                      85                  90                  95
   Thr Phe Gly Gly Gly Thr Lys Val Glu Ile Lys
               100                 105
<210> 61
   <211> 107
   <212> PRT
   <213> Artificial
<220>
   <223> artificial sequence
<400> 61
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                      80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys
               100                 105
<210> 62
   <211> 107
   <212> PRT
   <213> Artificial
<220>
   <223> artificial sequence
<400> 62
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Asp Pro Pro Leu
                   85                  90                  95
   Thr Phe Gly Gly Gly Thr Lys Val Glu Ile Lys
               100                 105
<210> 63
   <211> 107
   <212> PRT
   <213> Artificial
<220>
   <223> artificial sequence
<400> 63
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Arg Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Asp Pro Pro Leu
                   85                  90                  95
   Thr Phe Gly Gly Gly Thr Lys Val Glu Ile Lys
               100                 105
<210> 64
   <211> 107
   <212> PRT
   <213> Artificial
<220>
   <223> artificial sequence
<400> 64
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Arg Ala Asp Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Asp Pro Pro Leu
                   85                  90                  95
   Thr Phe Gly Gly Gly Thr Lys Val Glu Ile Lys
               100                 105
<210> 65
   <211> 325
   <212> PRT
   <213> Artificial
<220>
   <223> artificial sequence
<400> 65
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Cys Thr Leu Pro Pro Ser Gln Glu Glu Met Thr Lys
   225                 230                 235                 240
   Asn Gln Val Ser Leu Trp Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu
                   325
<210> 66
   <211> 325
   <212> PRT
   <213> Artificial
<220>
   <223> artificial sequence
<400> 66
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Pro Cys Pro Ala Pro
   100 105 110
   Glu Phe Leu Gly Gly Pro Ser Val Phe Leu Phe Pro Pro Lys Pro Lys
   115 120 125
   Asp Thr Leu Met Ile Ser Arg Thr Pro Glu Val Thr Cys Val Val Val
   130 135 140
   Asp Val Ser Gln Glu Asp Pro Glu Val Gln Phe Asn Trp Tyr Val Asp
   145 150 155 160
   Gly Val Glu Val His Asn Ala Lys Thr Lys Pro Arg Glu Glu Gln Phe
   165 170 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu
                   325
<210> 67
   <211> 325
   <212> PRT
   <213> Artificial
<220>
   <223> artificial sequence
<400> 67
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu
                   325
<210> 68
   <211> 325
   <212> PRT
   <213> Artificial
<220>
   <223> artificial sequence
<400> 68
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Lys Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Lys Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu
   325
<210> 69
   <211> 325
   <212> PRT
   <213> Artificial
<220>
   <223> artificial sequence
<400> 69
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Lys Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Pro
                   325
<210> 70
   <211> 325
   <212> PRT
   <213> Artificial
<220>
   <223> artificial sequence
<400> 70
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Lys Leu Thr Val Asp Lys Ser Arg Trp Gln Glu Gly Asn Val Phe Ser
   290 295 300
   Cys Ser Val Met His Glu Ala Leu His Asn Arg Tyr Thr Gln Lys Ser
   305 310 315 320
   Leu Ser Leu Ser Pro
   325
<210> 71
   <211> 325
   <212> PRT
   <213> Artificial
<220>
   <223> artificial sequence
<400> 71
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Cys Glu Met Thr Lys
   225                 230                 235                 240
   Asn Gln Val Ser Leu Ser Cys Ala Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Val Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu
                   325
<210> 72
   <211> 325
   <212> PRT
   <213> Artificial
<220>
   <223> artificial sequence
<400> 72
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Lys Leu Thr Val Asp Lys Ser Arg Trp Gln Glu Gly Asn Val Phe Ser
   290 295 300
   Cys Ser Val Met His Glu Ala Leu His Asn Arg Tyr Thr Gln Glu Ser
   305 310 315 320
   Leu Ser Leu Ser Pro
   325
<210> 73
   <211> 325
   <212> PRT
   <213> Artificial
<220>
   <223> artificial sequence
<400> 73
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Lys Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Glu Ser
   305                 310                 315                 320
   Leu Ser Leu Ser Pro
                   325
<210> 74
   <211> 107
   <212> PRT
   <213> Artificial
<220>
   <223> artificial sequence
<400> 74
<210> 75
   <211> 5
   <212> PRT
   <213> Artificial
<220>
   <223> artificial sequence
<400> 75
   Tyr Tyr Asp Met Ala
   1               5
<210> 76
   <211> 17
   <212> PRT
   <213> Artificial
<220>
   <223> artificial sequence
<400> 76
   Ser Ile Ser Pro Ser Gly Gly Ser Thr Tyr Tyr Arg His Ser Val Lys
   1 5 10 15
   Gly
<210> 77
   <211> 14
   <212> PRT
   <213> Artificial
<220>
   <223> artificial sequence
<400> 77
   Arg Ala Gly His Asn Leu Gly Ala Gly Trp Tyr Phe Asp Phe
   1 5 10
<210> 78
   <211> 5
   <212> PRT
   <213> Artificial
<220>
   <223> artificial sequence
<400> 78
   Tyr Tyr Asp Met Ala
   1 5
<210> 79
   <211> 17
   <212> PRT
   <213> Artificial
<220>
   <223> artificial sequence
<400> 79
   Ser Ile Ser Pro Ser Gly Gly Ser Thr Tyr Tyr Arg Arg Ser Val Lys
   1 5 10 15
   Gly
<210> 80
   <211> 14
   <212> PRT
   <213> Artificial
<220>
   <223> artificial sequence
<400> 80
   Arg Ala Gly His Asn Leu Gly Ala Gly Trp Tyr Phe Asp Phe
   1 5 10
<210> 81
   <211> 5
   <212> PRT
   <213> Artificial
<220>
   <223> artificial sequence
<400> 81
   Tyr Tyr Asp Met Ala
   1 5
<210> 82
   <211> 17
   <212> PRT
   <213> Artificial
<220>
   <223> artificial sequence
<400> 82
   Ser Ile Ser Pro Ser Gly Gly Ser Thr Tyr Tyr Arg Arg Ser Val Lys
   1 5 10 15
   Gly
<210> 83
   <211> 14
   <212> PRT
   <213> Artificial
<220>
   <223> artificial sequence
<400> 83
   Arg Ala Gly His Asn Phe Gly Ala Gly Trp Tyr Phe Asp Phe
   1 5 10
<210> 84
   <211> 5
   <212> PRT
   <213> Artificial
<220>
   <223> artificial sequence
<400> 84
   Tyr Tyr Asp Met Ala
   1 5
<210> 85
   <211> 17
   <212> PRT
   <213> Artificial
<220>
   <223> artificial sequence
<400> 85
   Ser Ile Ser Pro Ser Gly Gly Ser Thr Tyr Tyr Arg Arg Ser Val Lys
   1               5                   10                  15
   Gly
<210> 86
   <211> 14
   <212> PRT
   <213> Artificial
<220>
   <223> artificial sequence
<400> 86
   Arg Ala Gly His Asn Tyr Gly Ala Gly Trp Tyr Phe Asp Tyr
   1               5                   10
<210> 87
   <211> 5
   <212> PRT
   <213> Artificial
<220>
   <223> artificial sequence
<400> 87
   Tyr Tyr Asp Ile Asn
   1               5
<210> 88
   <211> 17
   <212> PRT
   <213> Artificial
<220>
   <223> artificial sequence
<400> 88
   Ser Ile Ser Pro Ser Gly Gly Ser Thr Tyr Tyr Arg Arg Ser Val Lys
   1               5                   10                  15
   Gly
<210> 89
   <211> 14
   <212> PRT
   <213> Artificial
<220>
   <223> artificial sequence
<400> 89
   Arg Ala Gly His Asn Tyr Gly Ala Gly Trp Tyr Phe Asp Tyr
   1 5 10
<210> 90
   <211> 5
   <212> PRT
   <213> Artificial
<220>
   <223> artificial sequence
<400> 90
   Tyr Tyr Asp Ile Asn
   1 5
<210> 91
   <211> 17
   <212> PRT
   <213> Artificial
<220>
   <223> artificial sequence
<400> 91
   Ser Ile Ser Pro Ser Gly Gln Ser Thr Tyr Tyr Arg Arg Glu Val Lys
   1 5 10 15
   Gly
<210> 92
   <211> 14
   <212> PRT
   <213> Artificial
<220>
   <223> artificial sequence
<400> 92
   Arg Ser Gly His Asn Tyr Gly Gly Gly Trp Tyr Phe Asp Tyr
   1 5 10
<210> 93
   <211> 5
   <212> PRT
   <213> Artificial
<220>
   <223> artificial sequence
<400> 93
   Tyr Tyr Asp Ile Gln
   1 5
<210> 94
   <211> 17
   <212> PRT
   <213> Artificial
<220>
   <223> artificial sequence
<400> 94
   Ser Ile Ser Pro Ser Gly Gln Ser Thr Tyr Tyr Arg Arg Glu Val Lys
   1 5 10 15
   Gly
<210> 95
   <211> 14
   <212> PRT
   <213> Artificial
<220>
   <223> artificial sequence
<400> 95
   Arg Ser Gly His Asn Tyr Gly Gly Gly Trp Tyr Phe Asp Tyr
   1 5 10
<210> 96
   <211> 5
   <212> PRT
   <213> Artificial
<220>
   <223> artificial sequence
<400> 96
   Tyr Tyr Asp Ile Ser
   1 5
<210> 97
   <211> 17
   <212> PRT
   <213> Artificial
<220>
   <223> artificial sequence
<400> 97
   Ser Ile Ser Pro Ser Gly Gln Ser Thr Tyr Tyr Arg Arg Glu Val Lys
   1 5 10 15
   Gly
<210> 98
   <211> 14
   <212> PRT
   <213> Artificial
<220>
   <223> artificial sequence
<400> 98
   Arg Ser Gly His Asn Phe Gly Gly Gly Trp Tyr Phe Asp Tyr
   1 5 10
<210> 99
   <211> 5
   <212> PRT
   <213> Artificial
<220>
   <223> artificial sequence
<400> 99
   Tyr Tyr Asp Ile Gln
   1 5
<210> 100
   <211> 17
   <212> PRT
   <213> Artificial
<220>
   <223> artificial sequence
<400> 100
   Ser Ile Ser Pro Ser Gly Gln Ser Thr Tyr Tyr Arg Arg Glu Val Lys
   1 5 10 15
   Gly
<210> 101
   <211> 14
   <212> PRT
   <213> Artificial
<220>
   <223> artificial sequence
<400> 101
   Arg Ser Gly Lys Ser Tyr Gly Gly Gly Trp Tyr Phe Asp Tyr
   1 5 10
<210> 102
   <211> 5
   <212> PRT
   <213> Artificial
<220>
   <223> artificial sequence
<400> 102
   Tyr Tyr Asp Ile Gln
   1 5
<210> 103
   <211> 17
   <212> PRT
   <213> Artificial
<220>
   <223> artificial sequence
<400> 103
   Ser Ile Ser Pro Ser Gly Gln Ser Thr Tyr Tyr Arg Arg Glu Val Lys
   1 5 10 15
   Gly
<210> 104
   <211> 14
   <212> PRT
   <213> Artificial
<220>
   <223> artificial sequence
<400> 104
   Arg Ser Gly Arg Glu Tyr Gly Gly Gly Trp Tyr Phe Asp Tyr
   1 5 10
<210> 105
   <211> 5
   <212> PRT
   <213> Artificial
<220>
   <223> artificial sequence
<400> 105
   Tyr Tyr Asp Ile Gln
   1 5
<210> 106
   <211> 17
   <212> PRT
   <213> Artificial
<220>
   <223> artificial sequence
<400> 106
   Ser Ile Ser Pro Ser Gly Gln Ser Thr Tyr Tyr Arg Arg Glu Val Lys
   1 5 10 15
   Gly
<210> 107
   <211> 14
   <212> PRT
   <213> Artificial
<220>
   <223> artificial sequence
<400> 107
   Arg Thr Gly Arg Glu Tyr Gly Gly Gly Trp Tyr Phe Asp Tyr
   1 5 10
<210> 108
   <211> 5
   <212> PRT
   <213> Artificial
<220>
   <223> artificial sequence
<400> 108
   Asp Asn Asn Met Asp
   1 5
<210> 109
   <211> 17
   <212> PRT
   <213> Artificial
<220>
   <223> artificial sequence
<400> 109
   Asp Ile Asn Thr Arg Ser Gly Gly Ser Ile Tyr Asn Glu Glu Phe Gln
   1 5 10 15
   Asp
<210> 110
   <211> 10
   <212> PRT
   <213> Artificial
<220>
   <223> artificial sequence
<400> 110
   Arg Lys Ser Tyr Gly Tyr His Leu Asp Glu
   1 5 10
<210> 111
   <211> 5
   <212> PRT
   <213> Artificial
<220>
   <223> artificial sequence
<400> 111
   Asp Asn Asn Met Asp
   1 5
<210> 112
   <211> 17
   <212> PRT
   <213> Artificial
<220>
   <223> artificial sequence
<400> 112
   Asp Ile Asn Thr Arg Ser Gly Gly Ser Ile Tyr Asn Glu Glu Phe Gln
   1 5 10 15
   Asp
<210> 113
   <211> 10
   <212> PRT
   <213> Artificial
<220>
   <223> artificial sequence
<400> 113
   Arg Lys Ser Tyr Gly Tyr Tyr Leu Asp Glu
   1 5 10
<210> 114
   <211> 5
   <212> PRT
   <213> Artificial
<220>
   <223> artificial sequence
<400> 114
   Asp Asn Asn Met Asp
   1 5
<210> 115
   <211> 17
   <212> PRT
   <213> Artificial
<220>
   <223> artificial sequence
<400> 115
   Asp Ile Asn Thr Arg Ser Gly Gly Ser Ile Tyr Asn Glu Glu Phe Gln
   1 5 10 15
   Asp
<210> 116
   <211> 10
   <212> PRT
   <213> Artificial
<220>
   <223> artificial sequence
<400> 116
   Arg Lys Ser Arg Gly Tyr His Leu Asp Glu
   1 5 10
<210> 117
   <211> 5
   <212> PRT
   <213> Artificial
<220>
   <223> artificial sequence
<400> 117
   Asp Asn Asn Met Asp
   1 5
<210> 118
   <211> 17
   <212> PRT
   <213> Artificial
<220>
   <223> artificial sequence
<400> 118
   Asp Ile Asn Thr Arg Ser Gly Gly Ser Ile Tyr Asn Glu Glu Phe Gln
   1 5 10 15
   Asp
<210> 119
   <211> 10
   <212> PRT
   <213> Artificial
<220>
   <223> artificial sequence
<400> 119
   Arg Lys Ser Tyr Gly Tyr Tyr Leu Asp Glu
   1 5 10
<210> 120
   <211> 5
   <212> PRT
   <213> Artificial
<220>
   <223> artificial sequence
<400> 120
   Asp Asn Asn Met His
   1 5
<210> 121
   <211> 17
   <212> PRT
   <213> Artificial
<220>
   <223> artificial sequence
<400> 121
   Asp Ile Asn Thr Arg Ser Gly Gly Ser Ile Tyr Asn Glu Glu Phe Gln
   1 5 10 15
   Asp
<210> 122
   <211> 10
   <212> PRT
   <213> Artificial
<220>
   <223> artificial sequence
<400> 122
   Arg Lys Ser Tyr Gly Tyr His Leu Asp Glu
   1 5 10
<210> 123
   <211> 5
   <212> PRT
   <213> Artificial
<220>
   <223> artificial sequence
<400> 123
   Asp Asn Asn Met Asp
   1 5
<210> 124
   <211> 17
   <212> PRT
   <213> Artificial
<220>
   <223> artificial sequence
<400> 124
   Asp Ile Asn Thr Arg Ser Gly Gly Ser Ile Tyr Asn Glu Glu Phe Gln
   1 5 10 15
   Asp
<210> 125
   <211> 10
   <212> PRT
   <213> Artificial
<220>
   <223> artificial sequence
<400> 125
   Arg Lys Ser Tyr Gly Asn His Leu Asp Glu
   1 5 10
<210> 126
   <211> 5
   <212> PRT
   <213> Artificial
<220>
   <223> artificial sequence
<400> 126
   Asp Asn Asn Met Asp
   1 5
<210> 127
   <211> 17
   <212> PRT
   <213> Artificial
<220>
   <223> artificial sequence
<400> 127
   Asp Ile Asn Thr Arg Ser Gly Gly Ser Ile Tyr Asn Glu Glu Phe Gln
   1 5 10 15
   Asp
<210> 128
   <211> 10
   <212> PRT
   <213> Artificial
<220>
   <223> artificial sequence
<400> 128
   Arg Lys Ser Tyr Gly Tyr Tyr Leu Asp Glu
   1 5 10
<210> 129
   <211> 5
   <212> PRT
   <213> Artificial
<220>
   <223> artificial sequence
<400> 129
   Asp Asn Asn Met Asp
   1 5
<210> 130
   <211> 17
   <212> PRT
   <213> Artificial
<220>
   <223> artificial sequence
<400> 130
   Asp Ile Asn Thr Arg Ser Gly Gly Ser Ile Tyr Asn Glu Glu Phe Gln
   1               5                   10                  15
   Asp
<210> 131
   <211> 10
   <212> PRT
   <213> Artificial
<220>
   <223> artificial sequence
<400> 131
   Arg Lys Ser Tyr Gly Tyr His Leu Asp Glu
   1               5                   10
<210> 132
   <211> 5
   <212> PRT
   <213> Artificial
<220>
   <223> artificial sequence
<400> 132
   Asp Asn Asn Met Asp
   1               5
<210> 133
   <211> 17
   <212> PRT
   <213> Artificial
<220>
   <223> artificial sequence
<400> 133
   Asp Ile Asn Thr Lys Ser Gly Gly Ser Ile Tyr Asn Glu Glu Phe Gln
   1               5                   10                  15
   Asp
<210> 134
   <211> 10
   <212> PRT
   <213> Artificial
<220>
   <223> artificial sequence
<400> 134
   Arg Gln Ser Tyr Gly Tyr His Leu Asp Glu
   1               5                   10
<210> 135
   <211> 5
   <212> PRT
   <213> Artificial
<220>
   <223> artificial sequence
<400> 135
   Asp Asn Asn Met Asp
   1               5
<210> 136
   <211> 17
   <212> PRT
   <213> Artificial
<220>
   <223> artificial sequence
<400> 136
   Asp Ile Asn Thr Arg Ser Gly Gly Ser Ile Tyr Asn Glu Glu Phe Gln
   1               5                   10                  15
   Asp
<210> 137
   <211> 10
   <212> PRT
   <213> Artificial
<220>
   <223> artificial sequence
<400> 137
   Arg Lys Ser Tyr Gly Tyr His Leu Asp Glu
   1               5                   10
<210> 138
   <211> 11
   <212> PRT
   <213> Artificial
<220>
   <223> artificial sequence
<400> 138
   Lys Ala Ser Gln Asn Ile Tyr Lys Asn Leu Ala
   1 5 10
<210> 139
   <211> 7
   <212> PRT
   <213> Artificial
<220>
   <223> artificial sequence
<400> 139
   Ser Ala Ser Tyr Arg Tyr Ser
   1 5
<210> 140
   <211> 8
   <212> PRT
   <213> Artificial
<220>
   <223> artificial sequence
<400> 140
   Gln Gln Tyr Tyr Ser Gly Leu Thr
   1 5
<210> 141
   <211> 11
   <212> PRT
   <213> Artificial
<220>
   <223> artificial sequence
<400> 141
   Lys Ala Ser Gln Asn Ile Tyr Lys Asn Leu Ala
   1 5 10
<210> 142
   <211> 7
   <212> PRT
   <213> Artificial
<220>
   <223> artificial sequence
<400> 142
   Ser Ala Ser Tyr Arg Tyr Ser
   1 5
<210> 143
   <211> 9
   <212> PRT
   <213> Artificial
<220>
   <223> artificial sequence
<400> 143
   Gln Gln Tyr Tyr Ser Pro Pro Leu Thr
   1 5
<210> 144
   <211> 11
   <212> PRT
   <213> Artificial
<220>
   <223> artificial sequence
<400> 144
   Lys Ala Ser Lys Asn Ile Glu Arg Asn Leu Ala
   1 5 10
<210> 145
   <211> 7
   <212> PRT
   <213> Artificial
<220>
   <223> artificial sequence
<400> 145
   Arg Ala Ser Arg Lys Glu Ser
   1 5
<210> 146
   <211> 9
   <212> PRT
   <213> Artificial
<220>
   <223> artificial sequence
<400> 146
   Gln Gln Tyr Ser Ser Pro Pro Leu Thr
   1 5
<210> 147
   <211> 11
   <212> PRT
   <213> Artificial
<220>
   <223> artificial sequence
<400> 147
   Lys Ala Ser Arg Asn Ile Glu Arg Asn Leu Ala
   1 5 10
<210> 148
   <211> 7
   <212> PRT
   <213> Artificial
<220>
   <223> artificial sequence
<400> 148
   Arg Ala Asp Arg Lys Glu Ser
   1 5
<210> 149
   <211> 9
   <212> PRT
   <213> Artificial
<220>
   <223> artificial sequence
<400> 149
   Gln Gln Tyr Ser Ser Pro Pro Leu Thr
   1 5
<210> 150
   <211> 11
   <212> PRT
   <213> Artificial
<220>
   <223> artificial sequence
<400> 150
   Lys Ala Ser Arg Asn Ile Glu Arg Asn Leu Ala
   1 5 10
<210> 151
   <211> 7
   <212> PRT
   <213> Artificial
<220>
   <223> artificial sequence
<400> 151
   Gln Ala Ser Arg Lys Glu Ser
   1 5
<210> 152
   <211> 9
   <212> PRT
   <213> Artificial
<220>
   <223> artificial sequence
<400> 152
   Gln Gln Tyr Ser Ser Pro Pro Leu Thr
   1 5
<210> 153
   <211> 11
   <212> PRT
   <213> Artificial
<220>
   <223> artificial sequence
<400> 153
   Lys Ala Ser Arg Asn Ile Glu Arg Gln Leu Ala
   1 5 10
<210> 154
   <211> 7
   <212> PRT
   <213> Artificial
<220>
   <223> artificial sequence
<400> 154
   Gln Ala Ser Arg Lys Glu Ser
   1 5
<210> 155
   <211> 9
   <212> PRT
   <213> Artificial
<220>
   <223> artificial sequence
<400> 155
   Gln Gln Tyr Ser Ser Pro Pro Leu Thr
   1 5
<210> 156
   <211> 11
   <212> PRT
   <213> Artificial
<220>
   <223> artificial sequence
<400> 156
   Lys Ala Ser Arg Asn Ile Glu Arg Gln Leu Ala
   1 5 10
<210> 157
   <211> 7
   <212> PRT
   <213> Artificial
<220>
   <223> artificial sequence
<400> 157
   Gln Ala Ser Arg Lys Glu Ser
   1 5
<210> 158
   <211> 9
   <212> PRT
   <213> Artificial
<220>
   <223> artificial sequence
<400> 158
   Gln Gln Tyr Ser Asp Pro Pro Leu Thr
   1 5
<210> 159
   <211> 11
   <212> PRT
   <213> Artificial
<220>
   <223> artificial sequence
<400> 159
   Lys Ala Ser Arg Asn Ile Glu Arg Gln Leu Ala
   1 5 10
<210> 160
   <211> 7
   <212> PRT
   <213> Artificial
<220>
   <223> artificial sequence
<400> 160
   Arg Ala Ser Arg Lys Glu Ser
   1 5
<210> 161
   <211> 9
   <212> PRT
   <213> Artificial
<220>
   <223> artificial sequence
<400> 161
   Gln Gln Tyr Ser Asp Pro Pro Leu Thr
   1 5
<210> 162
   <211> 11
   <212> PRT
   <213> Artificial
<220>
   <223> artificial sequence
<400> 162
   Lys Ala Ser Arg Asn Ile Glu Arg Gln Leu Ala
   1 5 10
<210> 163
   <211> 7
   <212> PRT
   <213> Artificial
<220>
   <223> artificial sequence
<400> 163
   Arg Ala Asp Arg Lys Glu Ser
   1 5
<210> 164
   <211> 9
   <212> PRT
   <213> Artificial
<220>
   <223> artificial sequence
<400> 164
   Gln Gln Tyr Ser Asp Pro Pro Leu Thr
   1 5
<210> 165
   <211> 118
   <212> PRT
   <213> Homo sapiens
<400> 165
   Glu Val Gln Leu Val Gln Ser Gly Ala Glu Val Gln Lys Pro Gly Ala
   1               5                   10                  15
   Ser Val Lys Val Ser Cys Lys Ala Ser Gly Tyr Thr Phe Thr Asp Tyr
               20                  25                  30
   Tyr Met His Trp Val Arg Gln Ala Pro Gly Glu Gly Leu Glu Trp Met
           35                  40                  45
   Gly Tyr Ile Asn Pro Ser Ser Gly Tyr Thr Lys Tyr Asn Arg Lys Phe
       50                  55                  60
   Arg Asp Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Gln Gly Tyr Tyr Leu Asp Tyr Trp Gly Glu Gly Thr
               100                 105                 110
   Thr Val Thr Val Ser Ser
           115
<210> 166
   <211> 119
   <212> PRT
   <213> Homo sapiens
<400> 166
   Glu Val Gln Leu Val Gln Ser Gly Ala Gln Val Lys Lys Pro Gly Ala
   1               5                   10                  15
   Ser Val Lys Val Ser Cys Lys Ala Ser Gly Tyr Thr Phe Ser Asp Asn
               20                  25                  30
   Asn Met Asp Trp Val Arg Gln Ala Pro Gly Lys Gly Leu Glu Trp Met
           35                  40                  45
   Gly Asp Ile Asn Thr Lys Ser Gly Gly Ser Ile Tyr Asn Gln Lys Phe
       50                  55                  60
   Lys Gly Arg Val Ile Met Thr Ile Asp Lys Ser Thr Gly Thr Ala Tyr
   65                  70                  75                  80
   Met Glu Leu Arg Ser Leu Arg Ser Asp Asp Thr Ala Ile Tyr Tyr Cys
                   85                  90                  95
   Ala Arg Arg Arg Ser Tyr Gly Tyr Tyr Phe Asp Tyr Trp Gly Arg Gly
               100                 105                 110
   Thr Leu Val Thr Val Ser Ser
           115
<210> 167
   <211> 106
   <212> PRT
   <213> Homo sapiens
<400> 167
   Asp Ile Val Met Thr Gln Ser Pro Ser Ser Leu Ser Ala Ser Val Gly
   1               5                   10                  15
   Asp Arg Val Thr Ile Thr Cys Lys Ala Ser Gln Asn Val Gly Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Ala Ser Gly Val Pro Ser Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Ser Asn Tyr Ile Thr
                   85                  90                  95
   Phe Gly Gln Gly Thr Lys Val Glu Ile Lys
               100                 105
<210> 168
   <211> 326
   <212> PRT
   <213> Homo sapiens
<400> 168
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305 310 315 320
   Leu Ser Leu Ser Leu Gly
   325
<210> 169
   <211> 107
   <212> PRT
   <213> Homo sapiens
<400> 169
<210> 170
   <211> 444
   <212> PRT
   <213> Artificial
<220>
   <223> artificial sequence
<400> 170
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Thr Met Thr Ile Asp Lys Ser Thr Gly Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Arg Ser Tyr Gly Tyr Tyr His Asp Glu Trp Gly Glu Gly
               100                 105                 110
   Thr Leu Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Lys Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Phe Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Gly Leu Pro Ser Ser Ile Glu Lys Thr Ile Ser
   325 330 335
   Lys Ala Lys Gly Gln Pro Arg Glu Pro Gln Val Tyr Thr Leu Pro Pro
   340 345 350
   Ser Gln Cys Glu Met Thr Lys Asn Gln Val Ser Leu Ser Cys Ala Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Val Ser Arg Leu Thr Val Asp Lys Ser Arg Trp
   405 410 415
   Gln Glu Gly Asn Val Phe Ser Cys Ser Val Met His Glu Ala Leu His
   420 425 430
   Asn His Tyr Thr Gln Lys Ser Leu Ser Leu Ser Leu
   435 440
<210> 171
   <211> 214
   <212> PRT
   <213> Artificial
<220>
   <223> artificial sequence
<400> 171
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Ser Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Tyr Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 172
   <211> 119
   <212> PRT
   <213> Artificial
<220>
   <223> artificial sequence
<400> 172
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Thr Met Thr Ile Asp Lys Ser Thr Gly Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Arg Ser Tyr Gly Tyr Tyr His Asp Glu Trp Gly Glu Gly
               100                 105                 110
   Thr Leu Val Thr Val Ser Ser
           115
<210> 173
   <211> 107
   <212> PRT
   <213> Artificial
<220>
   <223> artificial sequence
<400> 173
   Asp Ile Gln Met Thr Gln Ser Pro Ser Ser Leu Ser Ala Ser Val Gly
   1 5 10 15
   Asp Arg Val Thr Ile Thr Cys Lys Ala Ser Arg Asn Ile Glu Arg Asn
   20 25 30
   Leu Ala Trp Tyr Gln Gln Lys Pro Gly Gln Ala Pro Glu Leu Leu Ile
   35 40 45
   Tyr Ser Ala Ser Arg Lys Glu Ser Gly Val Pro Asp Arg Phe Ser Gly
   50 55 60
   Ser Arg Tyr Gly Thr Asp Phe Thr Leu Thr Ile Ser Ser Leu Gln Pro
   65 70 75 80
   Glu Asp Leu Ala Thr Tyr Tyr Cys Gln Gln Tyr Tyr Ser Pro Pro Leu
   85 90 95
   Thr Phe Gly Gly Gly Thr Lys Val Glu Ile Lys
   100 105
<210> 174
   <211> 5
   <212> PRT
   <213> Artificial
<220>
   <223> artificial sequence
<400> 174
   Asp Asn Asn Met Asp
   1 5
<210> 175
   <211> 17
   <212> PRT
   <213> Artificial
<220>
   <223> artificial sequence
<400> 175
   Asp Ile Asn Thr Arg Ser Gly Gly Ser Ile Tyr Asn Glu Glu Phe Gln
   1 5 10 15
   Asp
<210> 176
   <211> 10
   <212> PRT
   <213> Artificial
<220>
   <223> artificial sequence
<400> 176
   Arg Arg Ser Tyr Gly Tyr Tyr His Asp Glu
   1               5                   10
<210> 177
   <211> 11
   <212> PRT
   <213> Artificial
<220>
   <223> artificial sequence
<400> 177
   Lys Ala Ser Arg Asn Ile Glu Arg Asn Leu Ala
   1               5                   10
<210> 178
   <211> 7
   <212> PRT
   <213> Artificial
<220>
   <223> artificial sequence
<400> 178
   Ser Ala Ser Arg Lys Glu Ser
   1               5
<210> 179
   <211> 9
   <212> PRT
   <213> Artificial
<220>
   <223> artificial sequence
<400> 179
   Gln Gln Tyr Tyr Ser Pro Pro Leu Thr
   1               5